(19) Europäisches Patentamt
European Patent Office
Office européen des brevets

(11) EP 0 563 146 B1

# (12) EUROPÄISCHE PATENTSCHRIFT

(45) Veröffentlichungstag und Bekanntmachung des Hinweises auf die Patenterteilung: **06.10.1999 Patentblatt 1999/40**

(21) Anmeldenummer: **92901407.4**

(22) Anmeldetag: **18.12.1991**

(51) Int. Cl.$^6$: **C07D 213/61**, C07D 213/64, C07D 213/65, C07D 401/14, C07D 405/04, C07D 405/12, C07F 5/02, C07F 7/10, C09K 19/42, G02F 1/137

(86) Internationale Anmeldenummer: **PCT/EP91/02436**

(87) Internationale Veröffentlichungsnummer: **WO 92/11241 (09.07.1992 Gazette 1992/17)**

## (54) 2-FLUORPYRIDINE, VERFAHREN ZU IHRER HERSTELLUNG UND IHRE VERWENDUNG IN FLÜSSIGKRISTALLMISCHUNGEN

2-FLUOROPYRIDINE, PROCESS FOR PRODUCING IT AND ITS USE IN LIQUID CRYSTAL MIXTURES

2-FLUOROPYRIDINE, SON PROCEDE DE PRODUCTION ET SON UTILISATION DANS DES MELANGES DE CRISTAUX LIQUIDES

(84) Benannte Vertragsstaaten: **AT BE CH DE DK ES FR GB GR IT LI NL SE**

(30) Priorität: **19.12.1990 DE 4040575**
**09.04.1991 DE 4111461**

(43) Veröffentlichungstag der Anmeldung: **06.10.1993 Patentblatt 1993/40**

(60) Teilanmeldung: **99103925.6 / 0 930 301**

(73) Patentinhaber:
**HOECHST AKTIENGESELLSCHAFT**
**65926 Frankfurt am Main (DE)**

(72) Erfinder:
- **SCHLOSSER, Hubert**
  **D-6246 Glashütten (DE)**
- **WINGEN, Rainer**
  **D-6234 Hattersheim am Main (DE)**
- **ILLIAN, Gerd**
  **Nerimaku, Tokyo 176 (JP)**
- **ESCHER, Claus**
  **D-6109 Mühltal (DE)**

(74) Vertreter:
**Isenbruck, Günter, Dr. et al**
**Patent- und Rechtsanwälte Bardehle-Pagenberg-Dost-Altenburg-Geissler-Isenbruck**
**Theodor-Heuss-Anlage 12**
**68165 Mannheim (DE)**

(56) Entgegenhaltungen:
**EP-A- 0 336 619** **WO-A-91/04249**

EP 0 563 146 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die ungewöhnliche Kombination von anisotropem und fluidem Verhalten der Flüssigkristalle hat zu ihrer Verwendung in elektro-optischen Schalt- und Anzeigevorrichtungen geführt. Dabei können ihre elektrischen, magnetischen, elastischen und/oder thermischen Eigenschaften zu Orientierungsänderungen genutzt werden. Optische Effekte lassen sich beispielsweise mit Hilfe der Doppelberechnung, der Einlagerung dichroitisch absorbierender Farbstoffmoleküle ("guest-host mode") oder der Lichtstreuung erzielen.

Zur Erfüllung der ständig steigenden Praxisanforderungen auf den verschiedenen Anwendungsgebieten besteht laufend ein Bedarf an neuen verbesserten Flüssigkristall ("liquid crystal")-Mischungen und somit auch an einer Vielzahl mesogener Verbindungen unterschiedlicher Struktur. Dies gilt sowohl für die Gebiete, bei denen nematische LC-Phasen verwendet werden, als auch für solche mit smektischen LC-Phasen.

Auf besonderes Interesse sind in den letzten Jahren ferroelektrische flüssigkristalline Mischungen (FLC-Mischungen) gestoßen (siehe z.B. Lagerwall et al. "Ferroelectric Liquid Crystals for Display", SID Symposium, October Meeting 1985, San Diego, Ca. USA).

Für die praktische Verwendung von ferroelektrischen Flüssigkristallen in elektrooptischen Anzeigen werden chirale, geneigt-smektische Phasen wie z.B. $S_C$-Phasen benötigt [siehe R.B. Meyer, L.Liebert, L. Strzelecki und P.Keller, J. Physique 36, L-69 (1975)] , die über einen großen Temperaturbereich stabil sind. Dieses Ziel kann man mit Verbindungen erreichen, die selbst solche Phasen, z.B. $S_C$-Phasen, ausbilden oder aber, indem man nicht chirale geneigt-smektische Phasen ausbildende Verbindungen mit optisch aktiven Verbindungen dotiert [ siehe M. Brunet, Cl. Williams, Ann. Phys. 3, 237 (1978)].

Bei der Verwendung ferroelektrischer Flüssigkristallmischungen in elektro-optischen Bauelementen ist eine einheitliche planare Orientierung der Flüssigkristalle notwendig, um ein hohes Kontrastverhältnis zu erzielen. Es hat sich gezeigt, daß sich eine einheitliche planare Orientierung in der $S_C$-Phase erreichen läßt, wenn die Phasenfolge der Flüssigkristalllmischung mit abnehmender Temperatur lautet: Isotrop→nematisch→smektisch A→smektisch C (siehe z.B. K. Flatischler et al., Mol. Cryst. Sig. Cryst. 131, 21 (1985); T. Matsumoto et al., p. 468-470, Proc. of the 6th Int. Display Research Conf., Japan Display, 30. September - 2. October 1986, Tokyo, Japan; M.Murakami et al., ibid.p. 344-347).

Für ferroelektrische (chiral smektische) Flüssigkristallmischungen muß zusätzlich die Bedingung erfüllt sein,. daß die Ganghöhe (pitch) der Helix in der $S^*_C$-Phase groß, d. h. größer als 5 μm, und in der $N^*$-Phase sehr groß, d.h. größer als 10 μm bzw. unendlich ist.

Die optische Schaltzeit τ[μs] ferroelektrischer Flüssigkristallsysteme, die möglichst kurz sein soll, hängt von der Rotationsviskosität des Systems γ[mPas], der spontanen Polarisation $P_S$[nC/cm$^2$] und der elektrischen Feldstärke E[V/m] ab nach der Beziehung

$$\tau \approx \frac{\gamma}{P_S \cdot E}$$

Da die Feldstärke E durch den Elektrodenabstand im elektrooptischen Bauteil und durch die angelegte Spannung festgelegt ist, muß das ferroelektrische Anzeigemedium niedrigviskos sein und eine hohe spontane Polarisation aufweisen, damit eine kurze Schaltzeit erreicht wird.

Schließlich wird neben thermischer, chemischer und photochemischer Stabilität eine kleine optische Anisotropie Δn, vorzugsweise ≈ O,13, und eine geringe positive oder vorzugsweise negative dielektrische Anisotrophie Δε verlangt (siehe S.T. Lagerwall et al., "Ferroelectric Liquid Crystals for Displays" SID Symposium, Oct. Meeting 1985, San Diego, Ca, USA).

Die Gesamtheit dieser Forderungen ist nur mit Mischungen aus mehreren Komponenten zu erfüllen. Als Basis (oder Matrix) dienen dabei bevorzugt Verbindungen, die möglichst selbst bereits die gewünschte Phasenfolge I→N-$S_A$→$S_C$ aufweisen. Weitere Komponenten der Mischung werden oftmals zur Schmelzpunktserniedrigung und zur Verbreiterung der $S_C$- und meist auch N-Phase, zum Induzieren der optischen Aktivität, zur Pitch-Kompensation und zur Anpassung der optischen und dielektrischen Anisotropie zugesetzt, wobei aber beispielsweise die Rotationsviskosität möglichst nicht vergrößert werden soll.

Einzelne dieser Komponenten und auch bestimmte Mischungen sind bereits aus dem Stand der Technik bekannt. Da aber die Entwicklung, insbesondere von ferroelektrischen Flüssigkristallmischungen, noch in keiner Weise als abgeschlossen betrachtet werden kann, sind die Hersteller von Displays an unterschiedlichen Mischungen interessiert Dieses u.a. auch deshalb, weil erst das Zusammenwirken der flüssigkristallinen Mischungen mit den einzelnen Bauteilen der Anzeigevorrichtungen bzw. der Zellen (z.B. der Orientierungsschicht) Rückschlüsse auf die Qualität auch der flüssigkristallinen Mischungen zuläßt.

In EP-B 0 158 137 werden 4-Fluorpyrimidine als Verbindungen und als Mischungskomponenten allgemein

beschrieben. Sie weisen jedoch keine oder nur eine geringe Tendenz zur Ausbildung smektischer Phasen auf und finden daher Einsatz in nematischen Mischungen.

In DE-A 40 29 165 sowie in DE-A 40 30 582 werden 4-Fluorpyrimidine als Komponenten für ferroelektrische Flüssigkristallmischungen vorgestellt.

Desweiteren ist bekannt, daß Mono- und Difluorphenylverbindungen als Komponenten von Flüssigkristallmischungen verwendet werden Können (JP-A 2169-537; V.Reiffenrath, The Twelfth International Liquid Crystal Conference, Freiburg, 15.-19. August 1988). Diese Verbindungen haben jedoch zum Teil keine $S_C$-Phase. Ferner treten aufgrund fluorophober Wechselwirkungen häufig Mischbarkeitsprobleme mit strukturell unterschiedlichen Mischungskomponenten, z.B. Phenylpyrimidinen auf.

Pyridinderivate zeigen ebenfalls flüssigkristallines Verhalten unter Ausbildung einer $S_C$-Phase (T. Geelhaar, 1st International Symposium on Ferroelectric Liquid Crystal, Arcachon, 2.-23. September 1987, US 4,952,335). Eine in diesen Verbindungen häufig auftretende $S_I$-Phase beeinträchtigt jedoch deren Verwendung in ferroelektrischen Flüssigkristallmischungen.

Optisch aktive Pyridylethanolderivate sind aus der EP-A 0 336 619 bekannt.

Die vorliegende Erfindung betrifft 2-Fluorpyridin-Derivate und deren Verwendung als Komponenten für ferroelektrische Flüssigkristallmischungen.

2-Fluorpyridine sind aus den prioritätsälteren, nicht vorveröffentlichten WO-A 92/09576 und WO-A 91/04249, die gemäß Art. 54 (3 und 4) EPÜ Stand der Technik sind, bekannt, die dort offenbarten Verbindungen sind durch die Maßgabe in der folgenden Formel (I) ausgenommen.

Gegenstand der Erfindung ist eine Fluorpyridinverbindung der allgemeinen Formel I

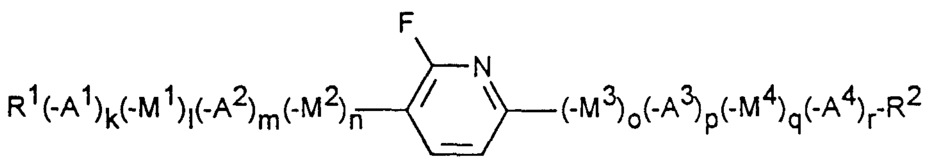

in der die Symbole folgende Bedeutung haben

$R^1$, $R^2$ unabhängig voneinander H, F, CN oder geradkettiges oder verzweigtes (mit oder ohne asymmetrisches C-Atom) Alkyl mit 1 bis 16 C-Atomen, wobei auch eine $-CH_2$-Gruppe durch -O-, -S-, -CO-, -CO-O-, -O-CO-, -O-CO-O-, -CH=CH-, -C≡C-, Δ oder $-Si(CH_3)_2$- ersetzt sein kann, oder eine der nachfolgenden chiralen Gruppen:

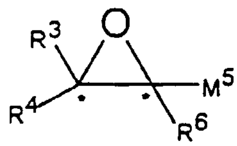 , 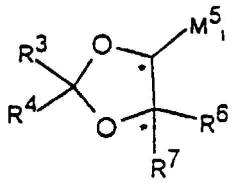 , 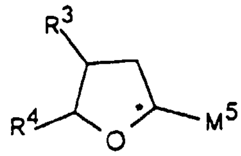 , 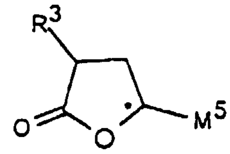

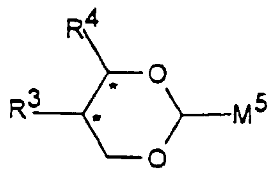 ,

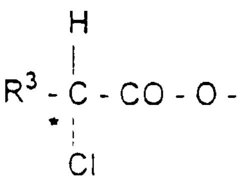 , 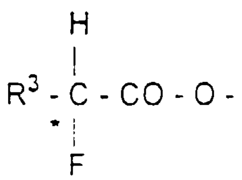 , 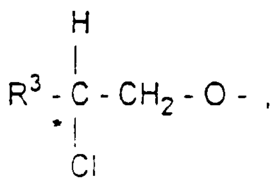 , 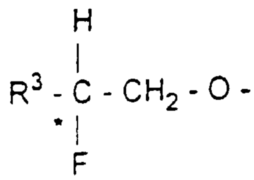 ,

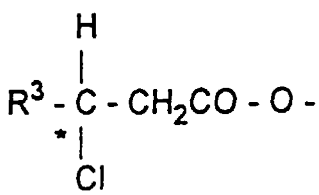 , 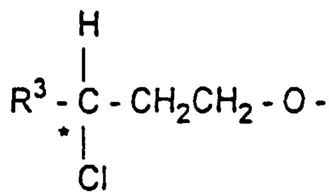 ,

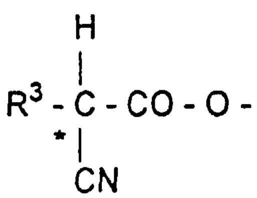 , 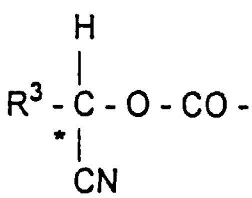

$A^1$, $A^2$, $A^3$, $A^4$ gleich oder verschieden 1,4-Phenylen, Pyrazin-2,5-diyl, Pyridazin-3,6-diyl, Pyridin-2,5-diyl, Pyrimidin-2,5-diyl, wobei ein oder zwei H-Atome durch F ersetzt sein können, trans-1,4-Cyclohexylen, (1,3,4)-Thiadiazol-2,5-diyl, 1,3-Dioxan-2,5-diyl, Naphthalin-2,6-diyl, Bicyclo[2.2.2.]octan-1,4-diyl oder 1,3-Dioxaborinan-2,5-diyl;
$M^1$, $M^2$, $M^3$, $M^4$ gleich oder verschieden -O-, -CO-, -CO-O-, -O-CO-, $-CH_2-O-$, $-O-CH_2-$, $-CH_2-CH_2-$, -CH=CH- oder -C≡C-;
$R^3$, $R^4$, $R^6$, $R^7$ unabhängig voneinander H oder geradkettiges oder verzweigtes Alkyl mit 1 bis 10 C-Atomen oder $R^3$ und $R^4$ zusammen auch $-(CH_2)_4-$ oder $-(CH_2)_5-$, wenn als Substituenten an ein Dioxolan System gebunden;
$M^5$ $-CH_2-O-$, -CO-O-, $-O-CH_2-$, -O-CO- oder eine Einfachbindung;
k, l, m, n, o, p, q, r Null oder Eins, unter der Bedingung, daß die Summe k+m+p+r kleiner 4 und größer Null ist;
mit der Maßgabe, daß

a) eine der beiden Gruppen $R^1$ und $R^2$ die Gruppe

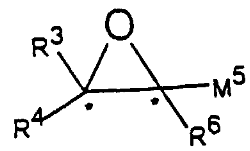

ist, oder

b) eine der Gruppen $A^1$ und $A^2$, $A^3$, $A^4$ 1,3-Dioxan-2,5-diyl,(1,3,4)-Thiadiazol-2,5-diyl oder 1,3-Dioxaborinan-2,5-diyl ist, oder

c) in einer der Gruppen $R^1$ und $R^2$ eine $-CH_2$-Gruppe durch $-Si(CH_3)$-ersetzt ist, oder

d) die Verbindung der Formel (I) ausgewählt ist aus

2-Fluor-6-(4-(4-octyloxyphenyl)phenyl)pyridin,
2-Fluor-3-octyloxy-6-(4-(4-octyloxyphenyl)phenyl)pyridin,
2-Fluor-3-hexyloxy-6-(4-(4-octylphenyl)phenyl)pyridin,
2-Fluor-3,6-di(4-octyloxyphenyl)pyridin,
2-Fluor-3-(4-heptylphenyl)-6(4-octyloxyphenyl)pyridin,
trans-4-Pentylcyclohexancarbonsäure-(2-fluor-6(4-octyloxyphenyl) pyridin-3-yl)ester,
2-Fluor-3-(4-(4-octylphenyl)phenyl)-6-(4-octyloxyphenyl)pyridin,
2-Fluor-3-octyloxy-6-[4-(4-octylphenyl)phenyl]pyridin.

Bevorzugte Fluorpyridinverbindungen ausgewählt aus

3(4-(Butyldimethylsilyl)butoxy-2-fluor-6-(4-(trans-4-pentylcyclohexyl)phenyl)pyridin,
2-Fluor-3-(5-octyl-1,3-dioxaborinan-2-yl)-6-(4-octyloxyphenyl)pyridin,
2-Fluor-3-(5-octyl-1,3-dioxan-2-yl)-6-(4-octyloxyphenyl)pyridin,
((2S,3S)-3-Pentyloxiran-2-yl)methyl-(2-fluor-6-(4-octyloxyphenyl)pyridin-3-yl)ether,
((2S,3S)-3-Pentyloxiran-2-yl)methyl-(4-(2-fluor-3-octyloxypyridin-6-yl) phenyl)ether,
((2S,3S)-3-Pentyloxiran-2-yl)methyl(2-fluor-6-(4-(trans-4-pentylcyclo hexyl)phenyl)pyridin-3-yl)ether.

Die erfindungsgemäßen Verbindungen sind chemisch und photochemisch stabil. Sie verfügen über niedrige Schmelzpunkte und im allgemeinen breite flüssigkristalline Phasen, insbesondere breite nematische, smektische A und smektische C-Phasen.

Aus flüssigkristallinen Verbindungen mit diesem Strukturelement lassen sich sowohl ferroelektrische Mischungen als auch nematische oder auch chiral nematische Mischungen herstellen, die für die Anwendung in elektrooptischen oder vollständig optischen Elementen, z.B. Anzeigeelementen, Schaltelementen, Lichtmodulatoren Elementen zur Bildbearbeitung, Signalverabeitung oder allgemein im Bereich der nichtlinearen Optik geeignet sind.

Ein besonderer Vorteil dieser Komponenten beruht auf ihrer negativen dielektrischen Anisotropie, die besonders günstig für die AC-Feld-Stabilisationen bei der Anwendung von ferroelektrischen Flüssigkristallen ist (siehe z.B. 1983 SID report von AT & T, JP-A 245142/1986, JP-A 246722/1986, JP-A 246723/1986).

Außerdem benötigt man für Displays, die sich vom ECB- bzw. CSH-Prinzip ableiten (siehe z-B. S. Yamauchi et al., SID 1969 Digest, Seite 378 oder M.F. Schiekel und K. Fahrensohn, Appl. Phys. Lett, S. 391 (1971)), nematische Mischungen mit möglichst großer negativer dielektrischer Anisotropie. Die Fluorpyridin-Derivate sind aufgrund ihrer breiten nematischen Phase, ihrer niedrigen Viskosität und ihrer großen negativen dielektrischen Anisotropie für diese nematischen Mischungen als Hauptkomponente oder als Beimischung geeignet.

Eine weitere Lösung der gestellten Aufgabe ist eine flüssigkristalline, insbesondere eine ferroelektrische flüssigkristalline Mischung, die mindestens eine Verbindung der allgemeinen Formel (I) enthält. Prinzipiell sind die genannten 2-Fluorpyridine jedoch ebenfalls als Komponenten für nematische Flüssigkristallmischungen sowie für den Einsatz in nematischen Displays geeignet.

Die Flüssigkristallmischungen bestehen im allgemeinen aus 2 bis 20, vorzugsweise 2 bis 15 Komponenten,

darunter mindestens eine der erfindungsgemäß beanspruchten Verbindungen. Die anderen Bestandteile werden vorzugsweise ausgewählt aus den bekannten Verbindungen mit nematischen, cholesterischen und/oder geneigt- smektischen Phasen, dazu gehörten beispielsweise Schiff'sche Basen, Biphenyle, Pyridine, Thiadizole, Difluorphenyle, Terphenyle, Phenyloyclohexane, Cyclohexylbiphenyle, Pyrimidine, Zimtsäureester, Cholesterinester sowie mehrkernige Ester von p-Alkylbenzoesäuren. Im allgemeinen liegen die im Handel erhältlichen Flüssigkristallmischungen bereits vor der Zugabe der erfindungsgemäßen Verbindung(en) als Gemische verschiedenere Komponenten vor, von denen mindestens eine mesogen ist.

Von dem oder den erfindungsgemäßen 2-Fluorpyridin-Derivaten enthalten die Flüssigkristallmischungen im allgemeinen 0,1 bis 70 Mol-%, bevorzugt 0,5 bis 50 Mol-%, insbesondere 1 bis 25 Mol-%.

Für die gebrauchsfertigen ferroelektrischen Flüssigkristallmischungen wurden die Werte für die spontane Polarisation $P_s[nC/cm^2]$, den Kontrast K und die optische Schaltzeit $\tau[\mu s]$ bestimmt, wobei alle Messungen bei einer Temperatur von 25°C vorgenomen wurden.

Die $P_s$-Werte werden nach der Methode von H. Diamant et al. (Rev. Sci. Instr., 28, 30, 1957) gemessen, wobei Meßzellen mit 2 µm Elektrodenabstand und geriebenem Polyimid als Orientierungsschicht verwendet werden. Zur Bestimmung von $\tau$ und K wird die Meßzelle auf dem Drehtisch eines Polarisationsmikroskops zwischen gekreuztem Analysator und Polarisator befestigt. Fur die Bestimmung des Kontrastes (K) wird die Meßzelle durch Drehen so positioniert, daß eine Photodiode minimalen Lichtdurchgang anzeigt (Dunkelzustand). Die Mikroskop-Beleuchtung wird so geregelt, daß die Photodiode für alle Zellen die gleiche Lichtintensität anzeigt. Nach einem Schaltvorgang ändert sich die Lichtintensität (Hellzustand) und der Kontrast wird aus dem Verhältnis der Lichtintensitäten dieser Zustände berechnet.

Zur Bestimmung von $\tau$ und des Schaltwinkels $\phi_{eff}$ wird durch Drehen des Tisches die Position des Tisches mit minimalem Lichtdurchgang für die beiden Schaltzustände in der Zelle bestimmt. Die Differenz der beiden Positionen am Drehtisch ist gleich dem doppelten effektiven Tiltwinkel. Mit Hilfe einer Photodiode erfolgt die Bestimmung der Schaltzeit $\tau$, indem die Anstiegszeit des Lichtsignals von 10 auf 90 % Signalhöhe gemessen wird. Die Schaltspannung besteht aus Rechteckpulsen und beträgt ± 10 V/µm.

Die Phasenumwandlungstemperaturen werden beim Aufheizen mit Hilfe eines Polarisationsmikroskops anhand der Texturänderungen bestimmt. Die Bestimmung des Schmelzpunkts wurde hingegen mit einem DSC-Gerät durchgeführt. Die Angabe der Phasenumwandlungstemperaturen zwischen den Phasen

| | |
|---|---|
| Nematisch | (N bzw. N*) |
| Smektisch-C | ($S_C$ bzw. $S_C$*) |
| Smektisch-A | ($S_A$ bzw. $S_A$*) |
| Kristallin | (X) |

erfolgt in °C und die Werte stehen zwischen den Phasenbezeichnungen in der Phasenfolge.

Flüssigkristalline Mischungen, die Verbindungen der allgemeinen Formel (I) enthalten, sind besonders für die Verwendung in elektrooptischen Schalt- und Anzeigevorrichtungen (Displays) geeignet. Schalt- und Anzeigevorrichtungen (LC-Displays) weisen u.a. folgende Bestandteile auf: ein flüssigkristallines Medium, Trägerplatten (z.B. aus Glas oder Kunststoff), beschichtet mit transparenten Elektroden, mindestens eine Orientierungsschicht, Abstandshalter, Kleberahmen, Polarisatoren sowie für Farbdisplays dünne Farbfilterschichten. Weitere mögliche Komponenten sind Antireflex-, Passivierungs-, Ausgleichs- und Sperrschichten sowie elektrisch-nichtlineare Elemente, wie z.B. Dünnschichttransistoren (TFT) und Metall-Isolator-Metall-(MIM)-Elemente. Im Detail ist der Aufbau von Flüssigkristalldisplays bereits in einschlägigen Monographien beschrieben (z.B. E. Kaneko, "Liquid Crystal TV Displays: Prinicples and Applications of Liquid Crystal Displays", KTK Scientific Publishers, 1987, Seiten 12-30 und 63-172).

Die Herstellung der erfindungsgemäßen Verbindungen kann durch die in den Schemata 1 bis 5 dargestellten Verfahren A und B erfolgen. Beide Verfahren unterscheiden sich prinzipiell dadurch, daß im Verfahren A ein Pyridinderivat, welches bereits die mesogene Grundstruktur einschließlich der Flügelgruppen enthält, durch geeignete Umsetzungen in Nachbarstellung zum Pyridin-Stickstoff fluoriert wird, während im Verfahren B ein bereits fluoriertes Pyridin durch geeignete Umsetzungen sukzessive mit weiteren mesogenen Einheiten und den Flügelgruppen versehen wird.

Beschrieben werden sowohl ein Verfahren zur Herstellung eines 2-Fluorpyridins der allgemeinen Formel (I) durch Fluorierung eines Pyridin-Derivates der Formel (A) am Pyridinring in 2-Position,

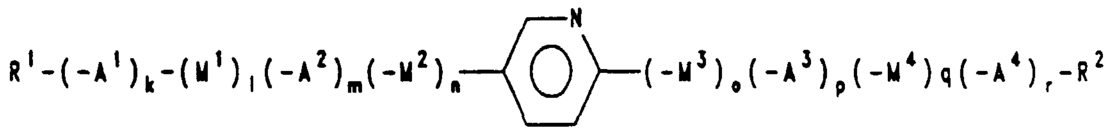

(A)

wie auch ein Verfahren, bei dem ausgehend von 2-Brom-6-fluorpyridin bzw. 2,6-Difluor-pyridin durch mehrstufige Umsetzung die Seitenketten

$R^1(-A^1)_k(-M^1)_l(-A^2)_m(-M^2)_n$- und
$-(M^3)_o-(A^3)_p-(M^4)_q-(A^4)_r-R^2$ in 3- bzw. 6-Position des Pyridinringes eingebracht werden.

Das in Schema 1 gezeigte Verfahren A geht von 2,5-disubstituierten Pyridinen der allgemeinen Formel (II) aus, die nach an sich literaturbekannten Methoden (z.B. C.S. Giam, J. Stout, Chem. Commun. 478 (1970); P. Doyle, R.R. Yates, Tetrahedron Lett. 3371 (1970); A.I. Pavljucenko et al., Z. Org. Chem. 22 (1986), 1061) dargestellt werden können. Die Pyridinderivate der Formel (II) lassen sich durch Umsetzung mit einer Percarbonsäure, z.B. Perameisensäure, Peressigsäure, Perbenzoesäure, 3-Chlorperbenzoesäure in das N-Oxid (Formel (III)) überführen. Die Verbindungen der Formel (III) können durch Behandlung mit einem Carbonsäureanhydrid und sich anschließender alkalischer Aufarbeitung in die Pyridone der Formel (IV) umgewandelt werden. Einen direkten Zugang zu den 2-Fluorpyridinderivaten der Formel (I) ausgehend von Pyridonen der Formel (IV) bietet die Umsetzung der letzteren mit einem Fluorierungsmittel wie z.B. Aminofluorosulfuranen (z.B. Diethylaminoschwefeltrifluorid) oder Schwefeltetrafluorid.

Alternativ können Pyridone der Formel (IV) unter Verwendung eines Halogenierungsmittels, wie z.B. Phosphortrichlorid, Phosphoroxytrichlorid, Phosphorpentachlorid und deren Brom- und Jod-Analoga, in die entsprechenden 2-Halogenpyridine der Formel (V) überführt werden. Der nucleophile Austausch der Halogensubstituenten gegen Fluor unter Zuhilfenahme eines Fluorid-Reagenzes wie z.B. Silberfluorid, Natriumfluorid, Kaliumfluorid oder Caesiumfluorid führt ebenfalls zu 2-Fluorpyridinen der Formel (I).

Schema 1

(Verfahren A)

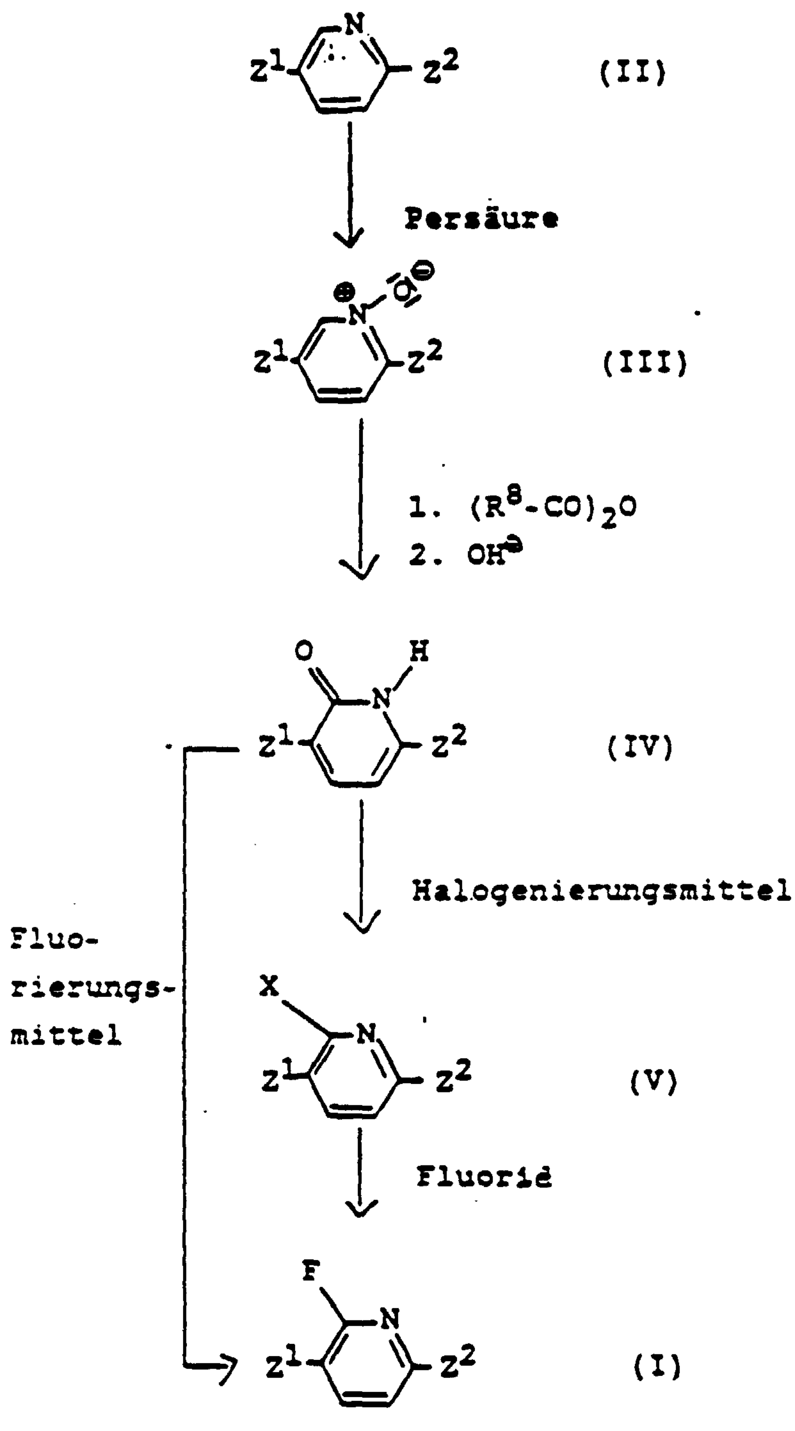


(II)

(III)

(IV)

(V)

(I)

$Z^1 = R^1(\text{-}A^1)_k(\text{-}M^1)_l(\text{-}A^2)_m(\text{-}M^2)_n\text{-}$

$Z^2 = (\text{-}M^3)_o(\text{-}A^3)_p(\text{-}M^4)_q(\text{-}A^4)_r\text{-}R^2$

X = Cl, Br, I

$R^8$ = geradkettiges oder verzweigtes Alkyl C-Atomen.

Die erste Ausgangsverbindung des in den Schemata 2 bis 5 skizzierten Verfahrens B ist das kommerziell erhältliche 2,6-Difluorpyridin (Formel (VI)). Der Austausch eines Fluorsubstituenten in (VI) gegen eine Gruppierung der allgemeinen Formel $Z^2$ $(-M^3)_o(-A^3)_p(-M^4)_q(-A^4)_r-R^2$ durch Umsetzung mit einer Metallverbindung von $Z^2$, z.B. einer Lithium-, Natrium-, Kalium- oder Magnesiumverbindung bei Temperaturen zwischen-40 und 100°C, insbesondere zwischen -10 und 70°C in einem inerten Reaktionsmedium, z.B. Diethylether, Tetrahydrofuran, 1,4-Dioxan, Ethylenglykoldiethylether oder Diethylenglykoldiethylether führt zu Verbindungen der Formel (IX).

Die zweite Ausgangsverbindung des Verfahrens B ist das ebenfalls kommerziell erhältliche 2,6-Dibrompyridin (Formel (VII)). Durch die Umsetzung von 2,6-Dibrompyridin mit 1 bis 3, insbesondere 1,5 bis 2,5 Mol-Äquivalenten eines Fluorid-Reagenzes, wie z.B. Silberfluorid, Natriumfluorid, Kaliumfluorid oder Caesiumfluorid, bei Temperaturen zwischen 50 und 250°C, insbesonders zwischen 100 und 200°C, und einem Druck zwischen 50 und 300 mm Hg, insbesonders zwischen 100 und 200 mm Hg, unter Verwendung katalytischer Mengen (1 bis 20 Mol-Prozent, insbesonders 5 bis 15 Mol-Prozent) eines Komplexbildners, wie z.B. 18-Krone-6, Dibenzo-18-Krone-6 oder 1,10-Diaza-4,7,13,16,21,24-hexaoxabicyclo[8.8.8]-hexacosan wird 2-Brom-6-fluorpyridin (VIII) erhalten.

Die Kreuzkupplung von Verbindung (VIII) mit metallorganischen Derivaten von $Z^2$, z.B. Grignard-, Lithium- und Zinkderivaten, sowie Boronsäuren von $Z^2$ unter Verwendung von Übergangsmetallkatalysatoren, z.B. Dichloro[1,3-bis(diphenylphosphino)propan]nickel[II] und Tetrakis(triphenylphosphin)palladium[0] bei Temperaturen zwischen -40 und $200^0$C, insbesondere zwischen -10 und 100°C, in Reaktionsmedien, wie z.B. Benzol/Ethanol/Wasser für die Umsetzung mit Boronsäuren von $Z^2$ und z.B. Diethylether oder Tetrahydrofuran für die Umsetzung mit Grignard-, Lithium- und Zinkderivaten von $Z^2$, liefert ebenfalls Verbindungen des Typs (IX).

2-Fluorpyridine des Typs (IX) können durch Behandlung mit einer Lithiumverbindung, wie Z.B. Lithiumalkylen oder Lithiumamiden, bei Temperaturen zwischen -40 und -100°C, insbesondere zwischen -60 und -80°C, in einem inerten Reaktionsmedium, z.B. Diethylether, Tetrahydrofuran oder Ethylenglykoldiethylether, in 2-Fluor-3-lithiumpyridine der Formel (X) überführt werden. 3-Lithiumpyridine der allgemeinen Formel (X) sind der Umsetzung mit elektrophilen Verbindungen zugänglich, wodurch entweder direkt oder über weitere Zwischenstufen (Verbindungen der Formeln (XI), (XII), (XIII), (XIV), (XV) und (XVI) 2-Fluorpyridine der Formel (I) erhalten werden können.

So führt die Reaktion von Verbindungen des Typs (X) mit Halogniden, Nitrilen Carbonsäurehalogeniden und Formylmethylderivaten von $Z^3$ bei Temperaturen zwischen -40 und -100°C, insbesondere zwischen-60 und -80°C, in einem inerten Reaktionsmedium, z.B. Diethylether, Tetrahydrofuran oder Ethylenglykoldiethylether, direkt zu 2-Fluorpyridinen der Formel (I). Olefinische 2-fluorpyridine (I) lassen sich durch Hydrierung der olefinischen Doppelbindung nach an sich literaturbekannten Methoden (siehe z.B. Houben-Weyl, Methoden der Organischen Chemie, Georg Thieme Verlag, Stuttgart) in gesättigte Spezies (I) umwandeln.

Die Umsetzung von 2-Fluor-3-lithiumpyridinen (X) mit Halogenen, z.B. Chlor, Brom oder Jod bei Temperaturen zwischen -40 und -100°C, insbesondere zwischen -60 und -80°C, in einem inerten Reaktionsmedium, z.B. Diethylether, Tetrahydrofuran oder Ethylenglykoldiethylether führt zu 2-Fluor-3-halogenpyridinen der Formel (XI). Durch Kreuzkupplung von Verbindungen des Typs (XI) mit metallorganischen Derivaten von $Z^1$, z.B. Grignard-, Lithium- und Zinkderivaten, sowie Boronsäuren von $Z^1$ unter Verwendung von Übergangsmetallkatalysatoren. z.B. Dichloro[1,3-bis(diphenylphosphino)propan]nickel[II] und Tetrakis(triphenylphosphin)palladium[0], bei Temperaturen zwischen -40 und 200°C, insbesondere zwischen -10 und 100°C, in Reaktionsmedien, wie z.B. Benzol/Ethanol/Wasser für die Umsetzung mit Boronsäuren von $Z^1$ und z.B. Diethylether oder Tetrahydrofuran für die Umsetzung mit Grignard-, Lithium- und Zinkdervaten von $Z^1$, erhält man 2-Fluorpyridine (I).

2-Fluor-3-lithiumpyridine (X) führen nach der Behandlung mit Kohlendioxid bei Temperaturen zwischen -40 und -100°C, insbesondere zwischen -60 und -80°C, in einem inerten Reaktionsmedium, z.B. Diethylether, Tetrahydrofuran oder Ethylenglykoldiethylether zu 2-Fluor-3-pyridincarbonsäuren der allgemeinen Formel (XII). Die Spezies (XII) können nach an sich literaturbekannten Methoden (siehe z.B. Houben-Weyl, Methoden der Organischen Chemie, Georg Thieme Verlag, Stuttgart), entweder direkt durch Veresterung mit Alkoholen von $Z^3$ unter Zuhilfenahme geeigneter Kondensationsmittel, z.B. Carbodiimiden, zu 2-Fluorpyridinen (I), oder nach Reduktion zu 2-Fluor-3-hydroxymethylpyridinen (XIII) mit geeigneten Reduktionsmitteln, z.B. komplexen Hydriden, durch Veresterung mit Carbonsäuren bzw. Carbonsäurehalogeniden von $Z^3$ oder Veretherung mit Alkoholen bzw. Halogeniden von $Z^3$ zu Verbindungen der Formel (I) umgesetzt werden.

Durch Reaktion von 2-Fluor-3-lithiumpyridinen (X) mit Ameisensäureamiden bei Temperaturen zwischen -40 und -100°C, insbesondere zwischen -60 und -80°C, in einem inerten Reaktionsmedium, z.B. Diethylether, Tetrahydrofuran oder Ethylenglykoldiethylether werden 2-Fluor-3-formylpyridine (XIV) erhalten, welche nach der sauer katalysierten Acetalisierung mit 2-$Z^4$-1,3-Propandiolen nach an sich literaturbekannten Methoden (siehe z.B. Houben-Weyl, Methoden der Organischen Chemie, Georg Thieme Verlag, Stuttgart) 2-Fluorpyridine des Typs (I) liefern.

Bei der sukzessiven Behandlung der 2-Fluor-lithiumpyridine (X) mit Borsäuretrialkylestern bei Temperaturen zwischen -40 und -100°C, insbesondere zwischen -60 und -80°C, und wäßriger Säure bei Temperaturen zwischen -10

und 50°C, insbesondere zwischen 10 und 30°C in einem inerten Reaktionsmedium, z.B. Diethylether, Tetrahydrofuran oder Ethylenglykoldiethylether, werden 2-Fluor-3-pyridinboronsäuren der Formel (XIV) erhalten.

Die Boronsäuren (XV) können Kupplungsreaktion mit Halogeniden von $Z^3$ unter Verwendung eines Übergangsmetallkatalysators, z.B. Tetrakis(triphenylphosphin)palladium[0] bei Temperaturen zwischen 30 und 200°C, insbesondere zwischen 50 und 100°C, in Reaktionsmedien, wie z.B. Benzol/Ethanol/Waser, zur Darstellung von Verbindungen des Typs (I) unterworden werden.

2-Fluorpyridine (I) werden aus den Boronsäuren (XV) desweiteren durch deren Veresterung mit 2-$Z^4$-1,3-Propandiolen nach an sich literaturbekannten Methoden (siehe z.B. Houben-Weyl, Methoden der Organischen Chemie, Georg Thieme Verlag, Stuttgart) erhalten.

Die Oxidation der Boronsäuren (XV) mit Peroxiden, z.B. Wasserstoffperoxid, bei Temperaturen zwischen 10 und 100°C, insbesondere zwischen 30 und 70°C, in Reaktionsmedien, wie z.B. Diethylether oder Tetrahydrofuran, führt zu den 2-Fluor-3-hydroxypyridinen (XVI), welche sich nach an sich literaturbekannten Methoden (siehe z.B. Houben-Weyl, Methoden der Organischen Chemie, Georg Thieme Verlag, Stuttgart) durch Veresterung mit Carbonsäuren bzw. Carbonsäurehalogeniden von $Z^3$ oder durch Veretherung mit Alkoholen bzw. Halogeniden von $Z^3$ In 2-Fluorpyridine der allgemeinen Formel (I) überführen lassen.

## Schema 2 (Verfahren B):

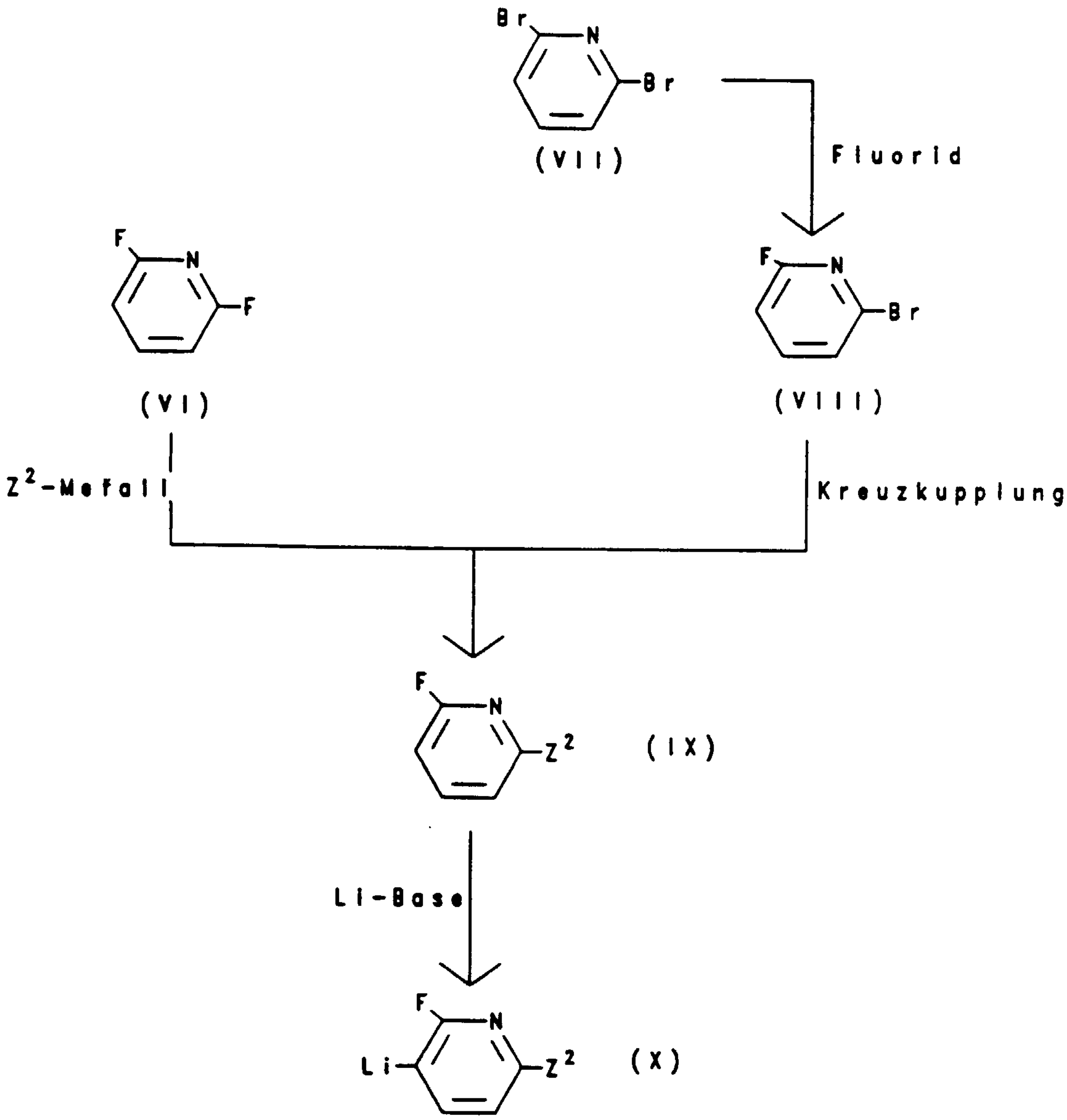

$Z^2$ siehe Schema 1

Schema 3 (Verfahren B):

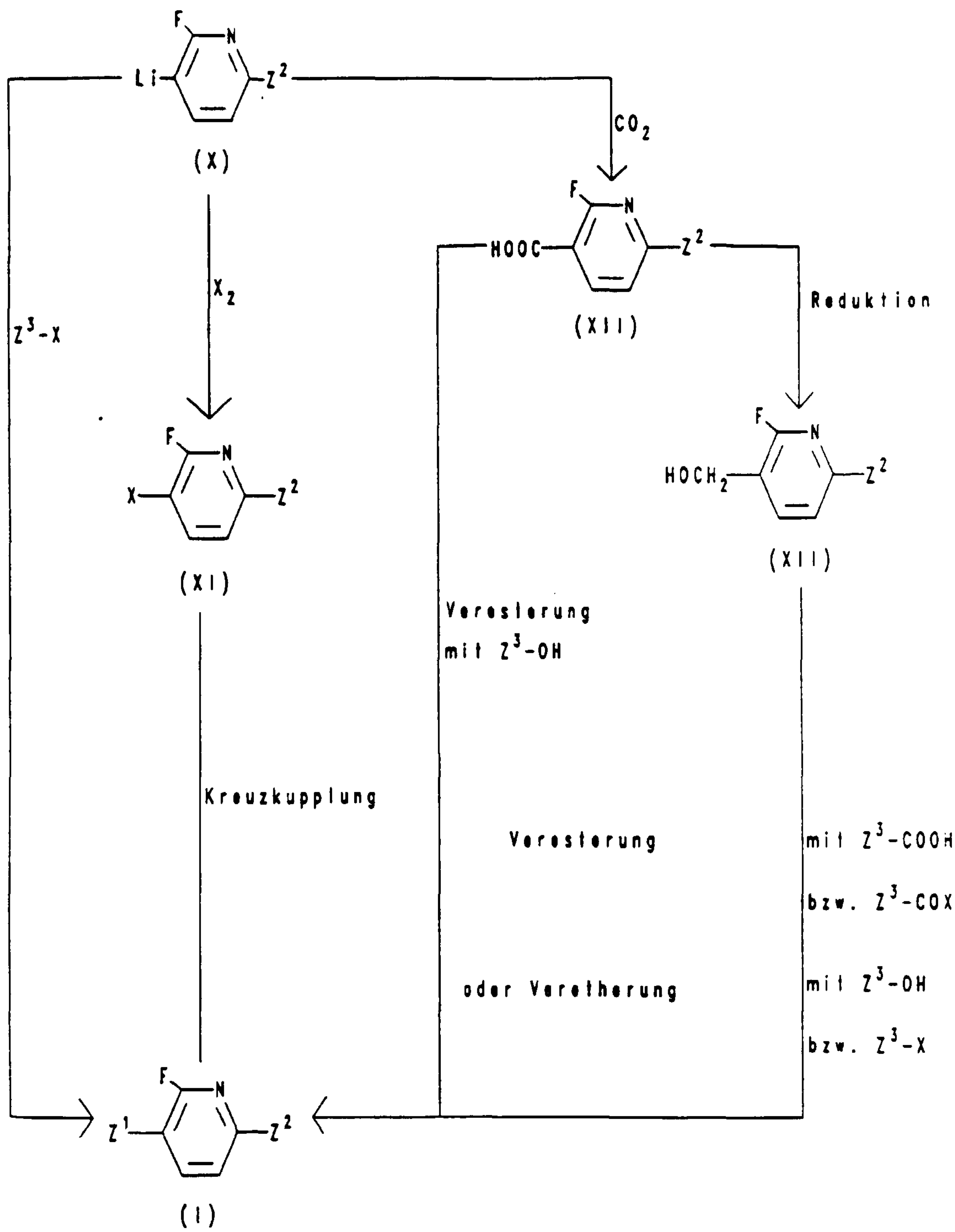


$Z^1$, $Z^2$, X siehe Schema 1

$Z^3 = R^1(-A^1)_k(-M^1)_l(-A^2)_m$-

Schema 4 (Verfahren B):

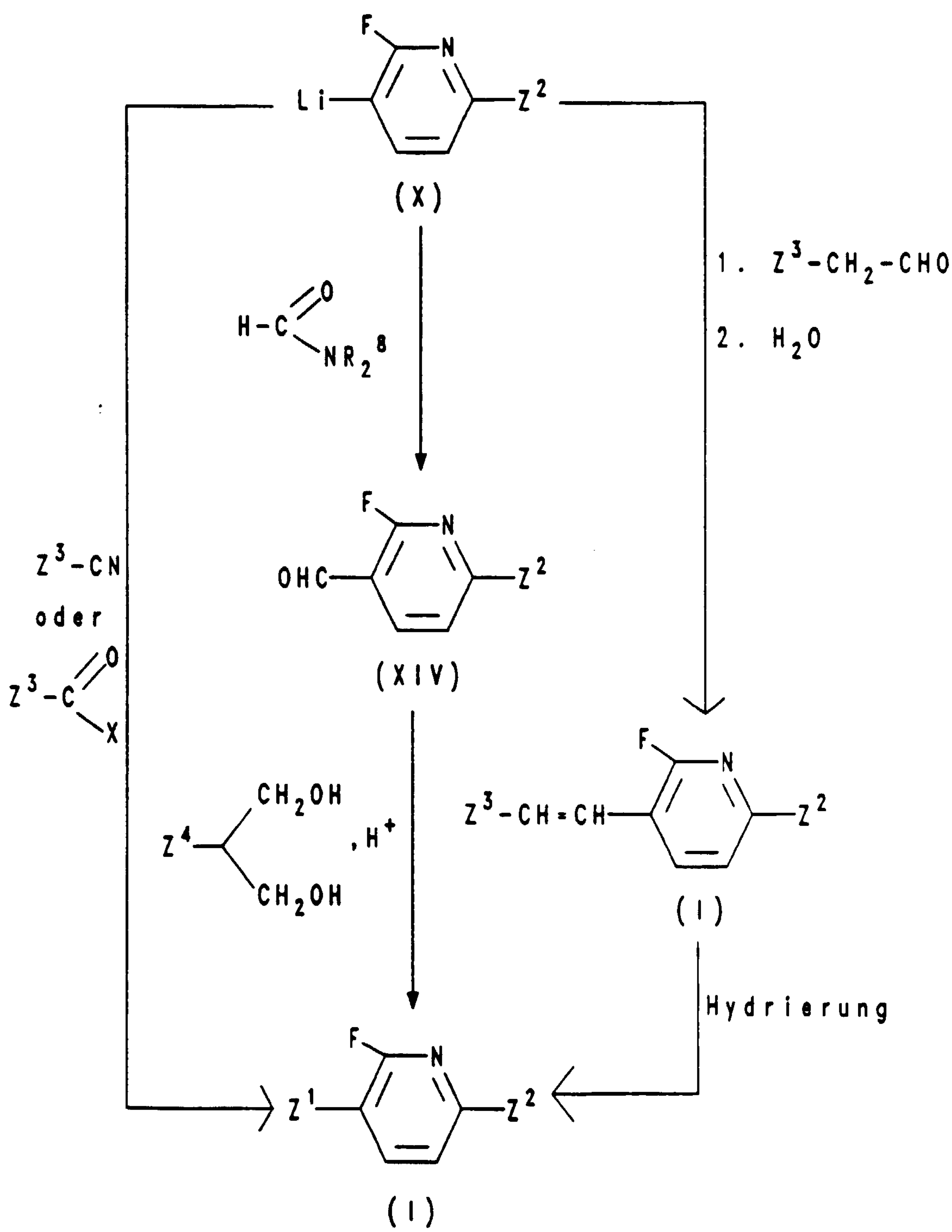


$Z^1$, $Z^2$, $Z^3$, $R^8$, X siehe Schemata 1 und 3
$Z^4 = R^1(-A^1)_k(-M^1)_l$-

Schema 5 (Verfahren B):

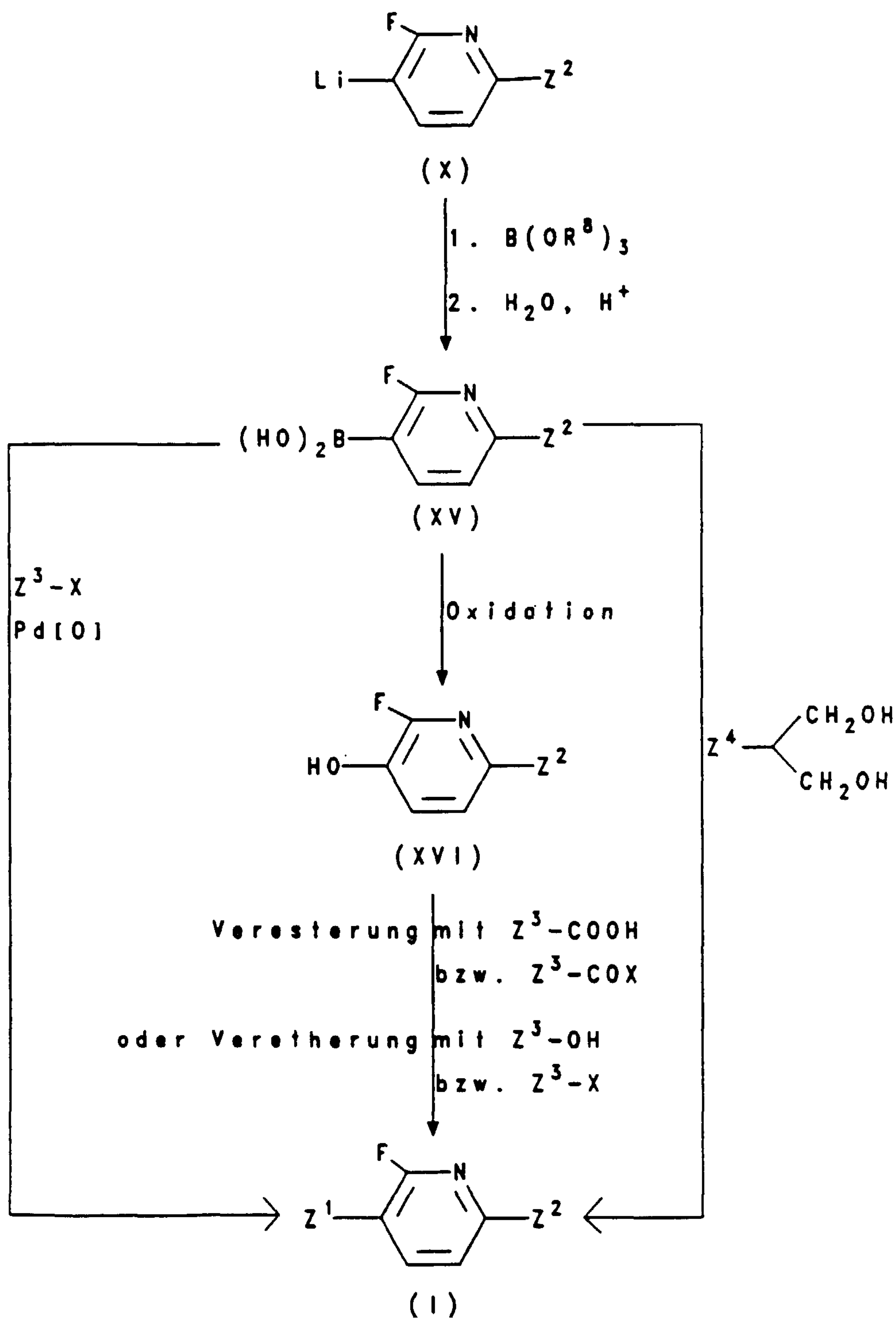


$Z^1$, $Z^2$, $Z^3$, $Z^3$, $R^8$, X siehe Schemata 1, 3 und 4

Die Erfindung wird durch die nachfolgenden Beispiele näher erläutert:

Referenz-Beispiel 1

2-Fluor-3-octyl-6-(4-octyloxyphenyl)pyridin

Darstellung nach Verfahren A:
Zu 3,23 g (8,2 mmol) 5-Octyl-2-(4-octyloxyphenyl)pyridin (hergestellt z.B. analog C.S. Giam, J. Staut, J. Chem. Soc., Chem. Commun. 478 (1970) aus Pyridin, 4-Octyloxyphenyllithium und 1-Bromoctan) in 30 ml Methylenchlorid wird bei einer Temperatur von 0 bis 5°C eine Lösung von 3,03 g (12,3 mmol) 3-Chlorperbenzosäure in 30 ml Methylenchlorid getropft. Nach einer Reaktionszeit von 3 h bei Raumtemperatur wird einmal mit 5 %iger Natriumcarbonat- und zweimal mit Natriumchloridlösung gewaschen, die organische Phase über Natriumsulfat getrocknet, abfiltriert und das Lösungsmittel abdestilliert. Nach chromatographischer Reinigung (Kleselgel/Methylenchlorid : Essigester = 8:2) des Rückstandes werden 2,20 g 5-Octyl-2-(4-octyloxphenyl)pyridin-N-oxid erhalten.

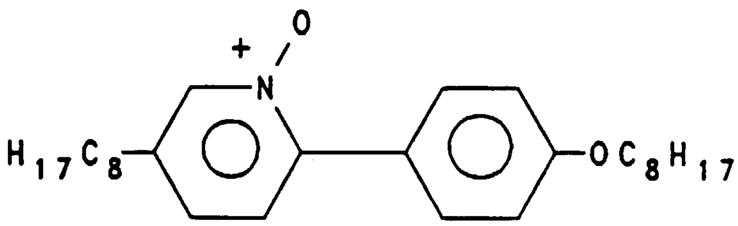

2,20 g (5,4 mmol) 5-Octyl-2-(4-octyloxyphenyl)pyridin-N-oxid werden in 30 ml Essigsäureanhydrid 4 h unter Rückfluß erhitzt. Anschließend wird das Essigsäureanhydrid abdestilliert und der Rückstand in 10 ml Methylenchlorid:Methanol (1:1) aufgenommen und 1 h mit methanolischer Kalilauge gerührt. Nach Waschen mit Wasser und konzentrierter Ammoniumchloridlösung, Trocknen über Natriumsulfat, Filtration, Abdestillation des Lösungsmittels und Umkristallisation aus n-Hexan:Essigester (8:2) werden 1,79 g 3-Octyl-6-(4-octyloxyphenyl)-1H-2-pyridon erhalten.

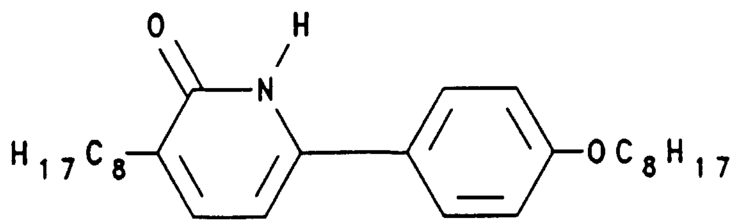

0,32 g (0,8 mmol) 3-Octyl-6-(4-octyloxyphenyl)-1H-2-pyridon werden mit 15 ml Phophoroxytrichlorid im Bombenrohr 3 h bei 200°C erhitzt. Das Reaktionsgemisch wird auf Eiswasser gegossen und mit Natriumcarbonat basisch gestellt. Nach Extraktion der wäßrigen Phase mit Methylenchlorid, Waschen der organischen Phase mit Natriumchloridlösung, Trocknen über Natriumsulfat, Filtration, Abdestillation des Lösungsmittels und Chromatographie (Kieselgel/Hexan : Essigester (8:2)) werden 0,22 g 2-Chlor-3-octyl-6-(4-octyloxyphenyl)pyridin erhalten.

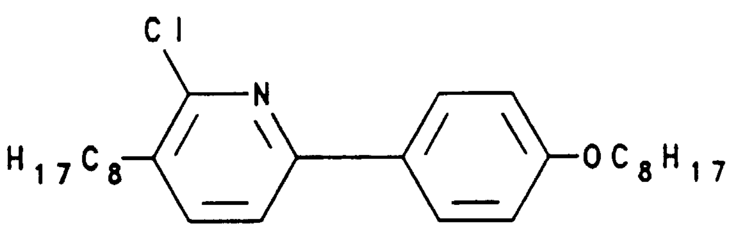

0,20 g (0,5 mmol) 2-Chlor-3-octyl-6-(4-octyloxyphenyl)pyridin werden mit 0,5 g Kaliumfluorid, 0,5 g Natriumfluorid und 0,02 g 18-Krone-6 im Bombenrohr 18 h bei 200°C erhitzt. Nach Aufnehmen des Reaktionsgemisches in

Ether/Wasser, Waschen der organischen Phase mit Wasser, Trocknen über Natriumsulfat, Filtration, Abdestillation des Lösungsmittels, chromatographischer Reinigung (Kieselgel/Hexan : Essigester (8:2)) und Umkristallisation aus Acetonitril werden 0,11 g 2-Fluor-3-octyl-6-(4-octyloxyphenyl)pyridin erhalten.

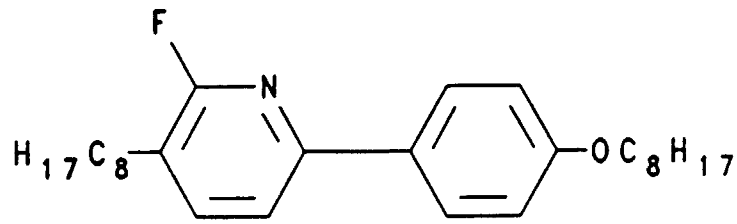

Die Verbindung zeigt folgende Phasenfolge:

X 58 $S_C$ 61 I

Referenz-Beispiel 2:

2-Fluor-3-octyl-6-(4-octyloxyphenyl)pyridin

Darstellung nach Verfahren B:
31,38 g (110,0 mmol) 1-Brom-4-octyloxybenzol in 80 ml Benzol und 81 ml (130,0 mmol) einer 1,6 molaren n-Butyllithiumlösung in Hexan werden unter Argon 4 h bei Raumtemperatur geführt. Das als weißer Niederschlag entstandene 4-Octyloxyphenyllithium wird unter Argon abfiltriert, zweimal mit 20 ml n-Hexan gewaschen, im Vakuum getrocknet und als Losung in 60 ml Tetrahydrofuran langsam bei 0°C unter Argon zu einer Lösung von 11,51 g (100,0 mmol) 2,6-Difluorpyridin in 80 ml Tetrahydrofuran getropft. Nach einstündiger Reaktionszeit bei 0°C wird mit Natriumchloridlösung versetzt, in Ether aufgenommen, die organische Phase mit Natriumchloridlösung gewaschen, über Natriumsulfat getrocknet, filtriert, vom Lösungsmittel befreit und chromatographisch (Kieselgel/Hexan: Essigester (9:1)) gereinigt. Es werden 4,00 g 2-Fluor-6-(4-octyloxyphenyl)pyridin erhalten.

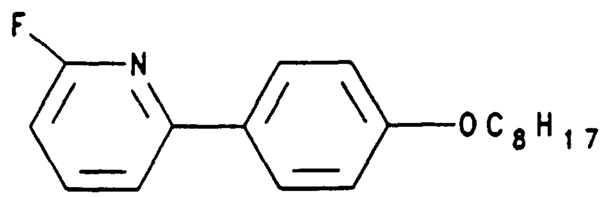

0,74 g (7,3 mmol) Diisopropylamin in 30 ml Tetrahydrofuran werden bei 0°C mit 4,56 ml (7,3 mmol) einer 1,6 molaren n-Butyllithiumlösung in Hexan für 1 h unter Argon gerührt. Nach Abkühlen auf -78°C werden 2,00 g (6,64 mmol) 2-Fluor-6-(4-octyloxyphenyl)pyridin in 30 ml Tetrahydrofuran so zugetropft, daß die Temperatur - 70°C nicht übersteigt. Nach 4 h bei -78°C werden 1,35 g (7,00 mmol) 1-Bromoctan in 10 ml Tetrahydrofuran ebenfalls unter Einhaltung einer Temperatur ≤-70°C zugetropft. Das Reaktionsgemisch wird über Nacht gerührt, wobei es sich auf Raumtemperatur erwärmt, mit 5 ml Wasser versetzt und zur Trockne eingeengt. Nach Aufnehmen in Ether wird zweimal mit Wasser gewaschen, über Natriumsulfat getrocknet, filtriert, das Lösungsmittel abdestilliert, chromatographisch gereinigt (Kieselgel/Hexan: Essigester (9:1)) und aus Acetonitril umkristallisiert. Es werden 0,10 g 2-Fluor-3-octyl-6-(4-octyloxyphenyl)pyridin erhalten, welches in seinen physikalischen Eigenschaften mit dem nach Beispiel 1 hergestellten Produkt übereinstimmt.

Referenz-Beispiel 3:

2-Fluor-3-octyloxy-6-(4-octyloxyphenyl)pyridin

Darstellung nach Verfahren B:
Zu 2,36 g (7,8 mmol) 2-Fluor-6-(4-octyloxyphenyl)-pyridin (hergestellt wie in Beispiel 2 beschrieben) in 180 mol Tetrahydrofuran werden bei -78°C 4,3 ml (8,6 mmol) einer 2-molaren Lithiumdiisopropylamidlösung in Tetrahydrofuran/Hexan/Ethylbenzol getropft und 4 h gerührt. Anschließend werden 2,95 g (17,2 mmol) Triisopropylborat in 10 ml Tetrahydrofuran bei -78°C zugetropft und das Reaktionsgemisch über Nacht gerührt, wobei es sich auf Raumtemperatur erwärmt. Nach Zugabe von 12,0 ml 10 %iger Salzsäure und 1 h Rühren bei Raumtemperatur wird mit Natriumchloridlösung versetzt, mit Ether extrahiert, die Etherphase mit Natriumchloridlösung gewaschen, über Natriumsulfat getrocknet, filtriert und eingeengt. Der Rückstand wird in 40 ml Ether aufgenommen und zum Sieden erhitzt. Es werden 8,03 g (23,5 mmol) einer 10 %igen Wasserstoffperoxidlösung zugetropft und 4 h unter Rückfluß gekocht. Nach Abtrennen der Etherphase wird die wäßrige Phase mit Ether ausgeschüttelt. Die vereinigten Etherphasen werden mit Natriumsulilösung ausgeschüttelt, mit Natriumchloridlösung gewaschen, über Natriumsulfat getrocknet, filtriert und zur Trockne eingeengt. Nach chromatographischer Reinigung (Kieselgel/Hexan: Essigester (7:3)) werden 1,13 g 2-Fluor-3-hydroxy-6-(4-octyloxyphenyl)pyridin erhalten.

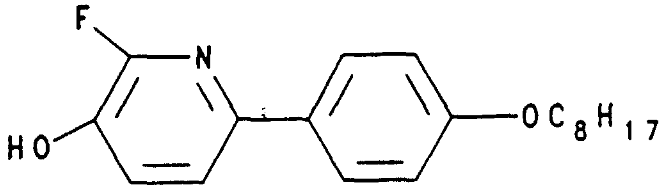

Zu 3,00 g (11,5 mmol) Triphenylphosphin in 50 ml Tetrahydrofuran werden bei 0°C 2,00 g (11,5 mmol) Azodicarbonsäurediethylester getropft und 30 min bei Raumtemperatur gerührt. Anschließend werden 2,42 g (7,7 mmol) 2-Fluor-3-hydroxy-6-(4-octyloxyphenyl)pyridin und 1,00 g (7,7 mmol) 1-Octanol zugegeben. Nach einer Reaktionszeit von 18 h bei Raumtemperatur wird das Lösungsmittel abdestilliert und der Rückstand chromatographisch (Kieselgel/Hexan: Essigester (95:5) gereinigt. Nach Umkristallisation aus Acetonitril werden 1,38 g 2-Fluor-3-octyloxy-6-(4-octyloxyphenyl)pyridin erhalten.

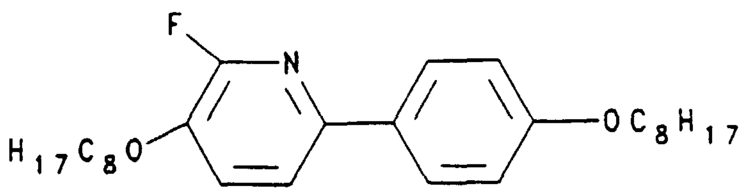

Die Verbindung zeigt die Phasenfolge:

X 81 $S_C$ 88 I

Referenz-Beispiel 4:

Octansäure-[2-fluor-6-(4-octyloxyphenyl)pyridin-3-yl]ester

Darstellung nach Verfahren B:
Zu 1,5 g (4,7 mmol) 2-Fluor-3-hydroxy-6-(4-octyloxyphenyl)pyridin (hergestellt wie in Beispiel 3 beschrieben) in 20 ml Pyridin werden bei 0°C 1,2 ml (7,1 mmol) Octansäurechlorid zugetropft und 3 h bei 0°C gerührt. Anschließend wird auf Eiswasser gegossen, abfiltriert und der Rückstand chromatographisch (Kieselgel/Hexan: Essigester (9:1) und durch Umkristallisation aus Acetonitril gereinigt. Es werden 1,34 g Octansäure-[2-fluor-6-(4-octyloxyphenyl)pyridin-3-yl]ester erhalten.

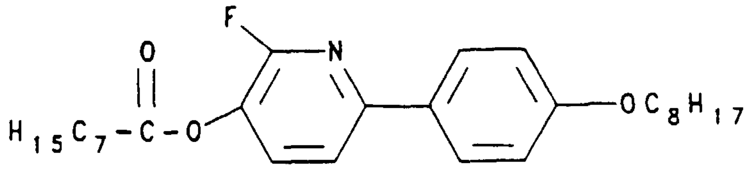

Die Verbindung zeigt die Phasenfolge:

X 63 $S_C$ 76 N 77 I

Referenz-Beispiel 5:

2-Fluor-6-octyloxy-3-(4-octyloxyphenyl)pyridin

Darstellung nach Verfahren B:
Lithiumoctanolat (das zuvor aus 13,02 g (100,00 mmol) 1-Octanol und 69 ml (110,00 mmol) einer 1,6 molaren n-Butyllithiumlösung in 40 ml Tetrahydrofuran bei 0°C hergestellt wurde) und 11,51 g (100,00 mmol) 2,6-Difluorpyridin werden in 40 ml Tetrahydrofuran 7 h unter Rückfluß erhitzt. Anschließend wird zwischen Natriumchloridlösung und Ether verteilt, die Etherphase zweimal mit Natriumchlorid gewaschen, über Natriumsulfat getrocknet, filtriert und vom Lösungsmittel befreit. Nach chromatographischer Reinigung (Kieselgel/Hexan:Essigester = 9:1) werden 13,48 g (59,80 mmol) 2-Fluor-6-octyloxypyridin erhalten.

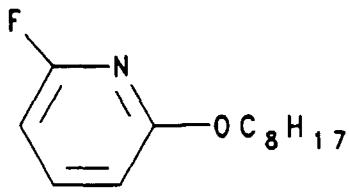

Zu 13,00 g (57,70 mmol) 2-Fluor-6-octyloxypyridin in 200 ml Tetrahydrofuran werden bei -78°C 34,62 ml (69,24 mmol) einer 2 molaren Lösung von Lithiumdiisopropylamid in Tetrahydrofuran/Hexan/Ethylbenzol getropft und 4 h gerührt. Anschließend werden 26,05 g (138,48 mmol) Triisopropylborat bei -78°C zugetropft und das Reaktionsgemisch über Nacht gerührt, wobei es sich auf Raumtemperatur erwärmt. Nach Zugabe von 80 ml 10 %iger Salzsäure und 1 h Rühren bei Raumtemperatur wird mit Natriumchloridlösung versetzt, mit Ether extrahiert, die Etherphase mit Natriumchloridlösung gewaschen, über Natriumsulfat getrocknet, filtriert und eingeengt. Nach Umkristallisation aus Acetonitril werden 9,03 g (33,55 mmol) 2-Fluor-6-octyloxypyridin-3-boronsäure erhalten.

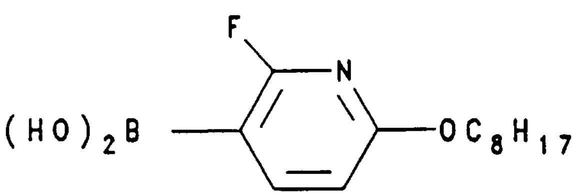

2,00 g (7,44 mmol) 2-Fluor-6-octyloxypyridin-3-boronsäure, 1,64 g (5,72 mmol) 1-Brom-4-octylorybenzol, 0,26 g (0,22 mmol) Tetratistriphenyllphosphinpalladium [O] und 11,16 mol (22,32 mmol) 2 Molare Natriumcarbonatlösung werden in 20 ml Benzol und 10 ml Ethanol 6 h unter Rückfluß erhitzt. Nach Verteilung des Reaktionsgemisches zwischen Wasser und Diethylether wird die organische Phase mit Wasser gewaschen, über Natriumsulfat getrocknet, filtriert und eingeengt. Nach Reinigung durch Chromatographie (Kieselgel/Hexan: Essigester = 95:5) und Umkristallisation aus Acetonitril werden 1,02 g 2-Fluor-6-octyloxy-3-(4-octyloxyphenyl)pyridin erhalten.

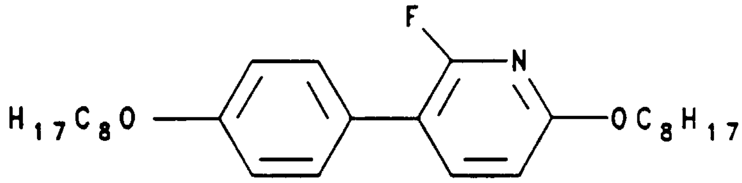

Die Verbindung zeigt die Phasenfolge:

X 39 $S_C$ 40 I

Beispiel 6:

2-Fluor-6-[4-(4-octyloxyphenyl)phenyl]pyridin

Darstellung nach Verfahren B:
24,00 g (66,60 mmol) 4-Brom-4'-octyloxybiphenyl in 500 ml Hexan und 160 ml Benzol werden mit 49 ml (78,70 mmol) einer 1,6 molaren n-Butyllithiumlösung in Hexan unter Argon 18 h bei Raumtemperatur gerührt. Danach wird das Reaktionsgemisch langsam bei 0°C unter Argon zu einer Lösung von 5,5 ml (60 mmol) 2,6-Difluorpyridin in 200 ml Tetrahydrofuran getropft. Nach fünfstündiger Reaktionszeit bie 0°C wird zur Trockne eingeengt, der Rückstand in Toluol aufgenommen und von festen Bestandteilen abfiltriert. Das Filtrat wird mit Wasser gewaschen, über Natriumsulfat getrocknet, filtriert und vom Lösungsmittel befreit. Nach Umkristallisation aus Hexan:Essigester = 8:2 werden 5,77 g 2-Fluor-6-[4-(4-octyloxyphenyl)phenyl]pyridin erhalten.

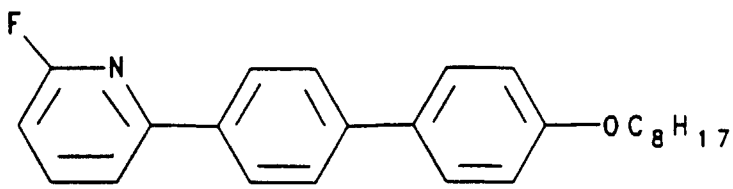

Die Verbindung zeigt die Phasenfolge:

X 148 $S_X$ 149 $S_C$ 150 $S_A$ 162 I

Beispiel 7:

2-Fluor-3-octyloxy-6-[4-(4-octyloxyphenyl)phenyl]pyridin

Darstellung nach Verfahren B:
Zu 5,00 g (13,25 mmol) 2-Fluor-6-[4-(4-octyloxyphenyl)phenyl]pyridin (hergestellt wie in Beispiel 6 beschrieben) in 1000 ml Tetrahydrofuran werden bei -78° C 7,95 ml (15,90 mmol) einer 2 molaren Lithiumdiisopropylamidlösung in Tetrahydrofuran/Hexan/Ethylbenzol getropft und 4 h geführt. Anschließend werden 5,98 (31,80 mmol) Triisopropylborat in 10 ml Tetrahydrofuran bei -78° C zugetropft und das Reaktionsgemisch über Nacht gerührt, wobei es sich auf Raumtemperatur erwärmt. Nach Zugabe von 20 ml 10 %iger Salzsäure und 1 h Rühren bei Raumtemperatur wird mit Natriumchloridlösung versetzt, mit Ether extrahiert, die Etherphase mit Natriumchlorid gewaschen, über Natriumsulfat getrocknet, filtriert und eingeengt. Der Rückstand wird in 200 ml Tetrahydrofuran aufgenommen, mit 40 ml 17,5 %iger Wasserstoffperoxidlösung 2 h bei 80°C gekocht und anschließend zwischen Wasser und Ether verteilt. Nach Waschen der Etherphase mit Natriumsulfitlösung und Wasser wird über Natriumsulfat getrocknet, filtriert, zur Trockne eingeengt und an Kieselgel mit Toluol:THF = 8:2 chromatographiert, wonach 1,7 g 2-Fluor-3-hydroxy-6-[4-(4-octyloxyphenyl)phenyl]pyridin erhalten werden.

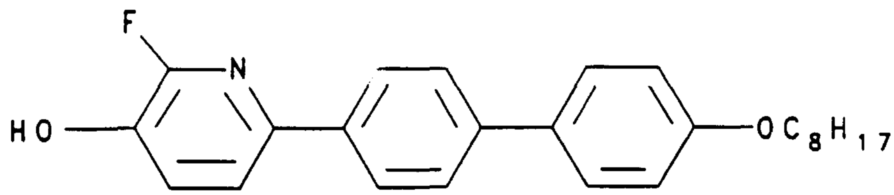

Zu 1,70 g (6,45 mmol) Triphenylphosphin in 100 ml Tetrahydrofuran werden bei 0°C 1,02 mol (6,45 mmol) Azodicarbonsäurediethylester getropft und 30 min bei Raumtemperatur gerührt. Anschließend werden 1,70 g (4,30 mmol) 2-Fluor-3-hydroxy-6-[4-(4-octyloxyphenyl)phenyl]pyridin und 0,56 g (4,30 mmol) 1-Octanol zugegeben. Nach 18-stündiger Reaktionszeit wird das ausgefallene Produkt abfiltriert und chromatographisch (Kieselgel/Dichlormethan) und durch Umkristallisation aus Acetonitril gereinigt. Es werden 1,13 g 2-Fluor-3-octyloxy-6-[4-(4-octyloxyphenyl)phenyl]pyridin erhalten.

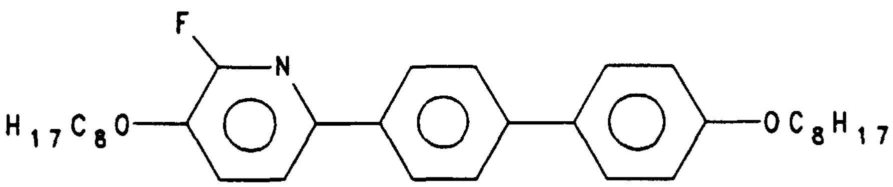

Die Verbindung zeigt folgende Phasenfolge:

X 144 $S_X$ 167 $S_C$ 208 I

Beispiel 8:

2-Fluor-3-octyloxy-6-[4-(4-octylphenyl)phenyl]pyridin

Die Darstellung erfolgt analog Beispiel 7.

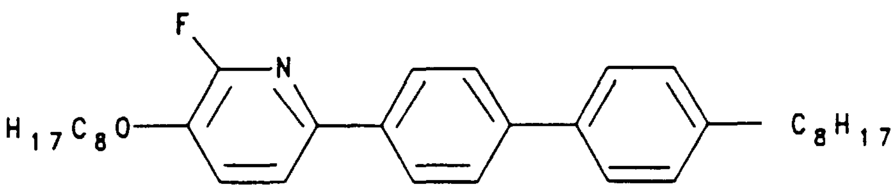

Die Verbindung zeigt folgende Phasenfolge:

X 98 $S_3$ 122 $S_2$ 145 $S_C$ 184 I

Beispiel 9:

2-Fluor-3-hexyloxy-6-[4-(4-octylphenyl)phenyl]pyridin

Die Darstellung erfolgt analog Beispiel 7.

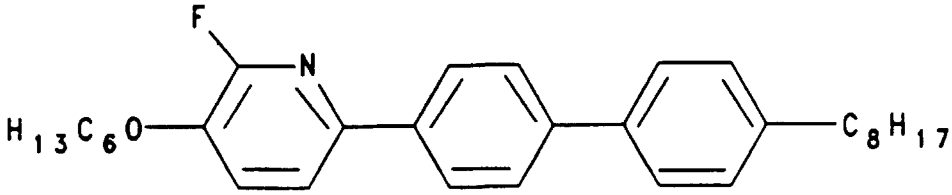

Die Verbindung zeigt die Phasenfolge:

X 80 $S_3$ 142 $S_2$ 155 $S_C$ 189 I

Beispiel 10:

2-Fluor-3,6-di(4-octyloxyphenyl)pyridin

Darstellung nach Verfahren B:
8,00 ml (56,80 mmol) Diisopropylamin in 10 ml Tetrahydrofuran werden bei 0°C eine Stunde mit 35,70 ml (56,80 mmol) einer 1,6 molaren n-Butyllithiumlösung in Hexan gerührt. Nach Abkühlen auf -78°C werden 8,56 g (28,40 mmol) 2-Fluor-6-(4-ocyloxyphenyl)pyridin (hergestellt wie in Beispiel 2 beschrieben) in 1000 ml Tetrahydrofuran zugetropft und 4 h bei -78°C gerührt. Anschließend werden 21,25 g (113,00 mmol) Triisopropylborat bei -78°C zugetropft und das Reaktionsgemisch über Nacht gerührt, wobei es sich auf Raumtemperatur erwärmt. Nach Zugabe von 100 ml 10 %iger Salzsäure und einstündigem Rühren bei Raumtemperatur wird mit Natriumchloridlösung versetzt, mit Ether extrahiert, die Etherphase mit Natriumchloridlösung gewaschen, über Natriumsulfat getrocknet, filtriert und eingeengt. Nach Umkristallisation aus Acetonitril wreden 7,70 g 2-Fluor-6-(4-octyloxyphenyl)pyridin-3-boronsäure erhalten.

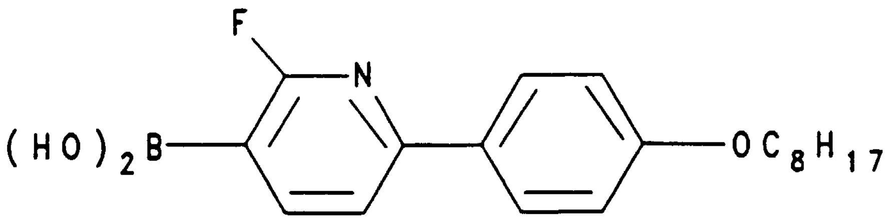

3,35 g (10,00 mmol) 2-Fluor-6-(4-octyloxyphenyl)pyridin-3-boronsäure in 45 ml Ethanol werden mit 2,85 g (10,00 mmol) 1-Brom-4-octyloxybenzol und 0,37 g (0,31 mmol) Tetrakistriphenylphosphinpalladium in 60 ml Benzol sowie 3,18 g (30,00 mmol) Natriumcarbonat in 12 ml Wasser 20 h unter Rückfluß erhitzt. Nach Verteilung des Reaktionsgemisches zwischen Wasser und Dichlormethan wird die organische Phase mit Natriumchloridlösung gewaschen, über Natriumsulfat getrocknet, filtriert und eingeengt. Nach Reinigung durch Chromatographie (Kieselgel/Dichlormethan) und Umkristallisation aus Acetonitril werden 2,5 g 2-Fluor-3,6-di(4-octyloxyphenyl)pyridin erhalten.

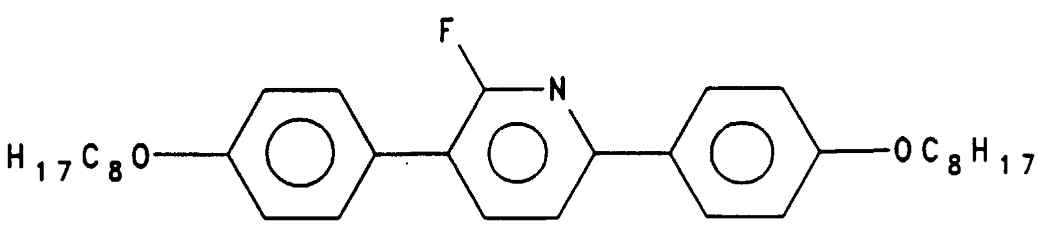

Die Verbindung zeigt folgende Phasenfolge:

X 105 $S_C$ 176 N 182 I

Diese Verbindung unterscheidet sich von der Beispielverbindung 7 durch die Stellung der Fluorpyridingruppe im Ringsystem. Die mittlere Stellung der Fluorpyridingruppe ist vorteilhaft, da die oben genannte Beispielverbindung im Vergleich zur Beispielverbindung 4 einen um 38° C niedrigeren Schmelzpunkt aufweist. Außerdem kann auch noch eine nematische Phase beobachtet werden und die $S_C$-Phasenbreite ist fast doppelt so groß.

Beispiel 11:

2-Fluor-3-(4-heptylphenyl)-6-(4-octyloxyphenyl)pyridin

Die Darstellung erfolgt analog Beispiel 10.

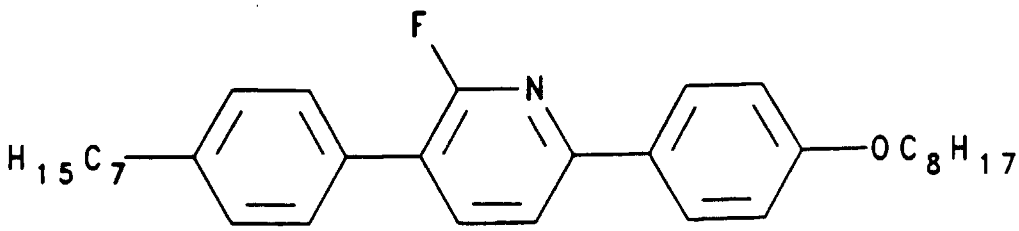

Die Verbindung zeigt die Phasenfolge:

X 58 $S_3$ 57 $S_2$ 65 $S_C$ 157 N 164 I

Referenz-Beispiel 12:

2-Fluor-6-octyloxy-3-[4-(4-octyloxyphenyl)phenyl]pyridin

Darstellung nach Verfahren B:
2,10 g (7,50 mmol) 2-Fluor-6-octyloxypyridin-3-boronsäure (hergestellt wie in Beispiel 5 beschrieben) in 35 ml Ethanol werden mit 2,70 g (7,50 mmol) 4-Brom-4*-octyloxybiphenyl und 0,29 g (0,24 mmol) Tetrakistriphenylphosphinpalladium in 46 ml Benzol sowie 2,5 g (23,40 mmol) Natriumcarbonat in 11 ml Wasser 20 h unter Rückfluß erhitzt. Nach Verteilung des Reaktionsgemisches zwischen Wasser und Dichlormethan wird die organische Phase mit Natriumchloridlösung gewaschen, über Natriumsulfat getrocknet, filtriert und eingeengt. Nach Reinigung durch Chromatographie (Kieselgel/Dichlormethan) und Umkristallisation aus Acetonitril werden 0,50 g 2-Fluor-6-octyloxy-3-[4-(4-octyloxyphenyl)phenyl]pyridin erhalten.

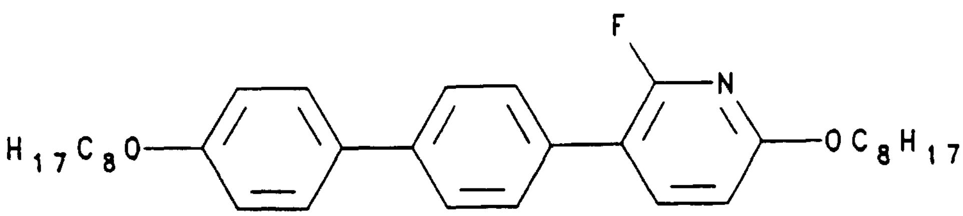

Die Verbindung zeigt folgende Phasenfolge:

X 87 $S_X$ 116 $S_C$ 158 $S_A$ 163 I

Referenz-Beispiel 13:

2-Fluor-6-hexyloxy-3-[4-(4-octyloxyphenyl)phenyl]pyridin

Die Darstellung erfolgt analog Beispiel 12.

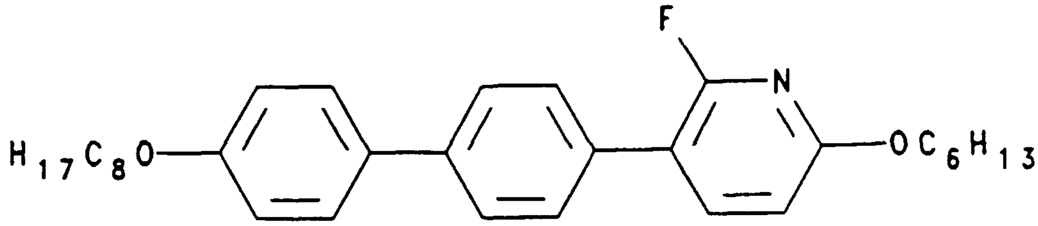

Die Verbindung zeigt die Phasenfolge:

X 85 $S_4$ 93 $S_3$ 119 $S_C$ 161 $S_A$ 169 I

Referenz-Beispiel 14:

2-Fluor-6-octyloxy-3-[4-(4-octylphenyl)phenyl]pyridin

Die Darstellung erfolgt analog Beispiel 12.

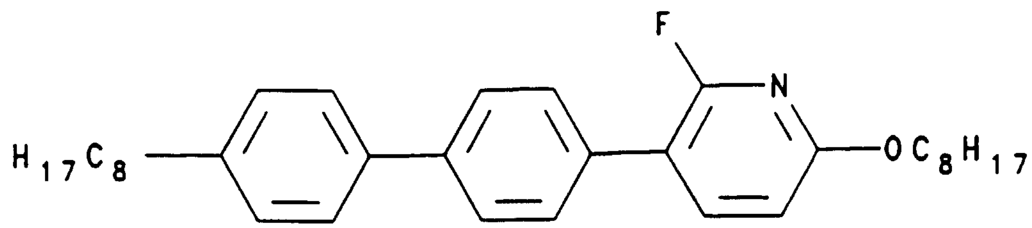

Die Verbindung zeigt die Phasenfolge:

X 62 $S_X$ 96 $S_C$ 116 $S_A$ 133 I

Referenz-Beispiel 15:

2-Fluor-6-hexyloxy-[4-(4-octylphenyl)phenyl]pyridin

Die Darstellung erfolgt analog Beispiel 12.

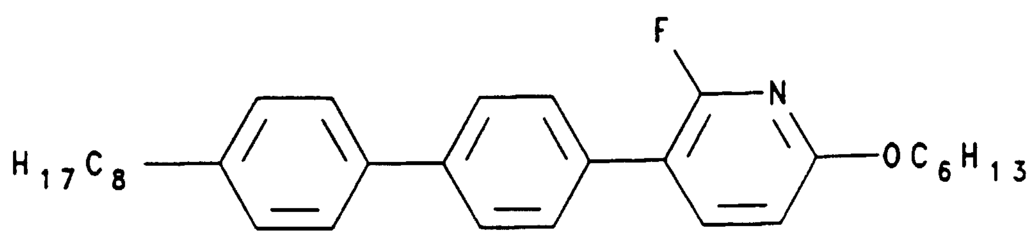

Die Verbindung zeigt die Phasenfolge:

X 44 $S_4$ 92 $S_3$ 99 $S_C$ 118 $S_A$ 138 I

Die Tendenz der 2-Fluorpyridin-Verbindungen zur Ausbildung breiter $S_C$-Phasen wird durch die Beispiele 12 bis 15 verdeutlicht.

Referenz-Beispiel 16:

1,4-Di(2-Fluor-6-octyloxypyridin-3-yl)benzol

Darstellung nach Verfahren B:
0,87 g (3,7 mmol) 1,4-Dibrombenzol, 2,00 g (7,4 mmol) 2-Fluor-6-octyloxypyridin-4-boronsäure (hergestellt wie in Beispiel 5 beschrieben), 0,56 g (0,46 mmol) Tetrakistriphenylphosphinpalladium[O] und 5 g (46,1 mmol) Natriumcarbonat in 50 ml Benzol, 35 ml Ethanol und 20 ml Wasser werden 18 h unter Rückfluß erhitzt. Nach Verteilung des Reaktionsgemisches zwischen Wasser und Dichlormethan wird die organische Phase mit Natriumchloridlösung gewaschen, über Natriumsulfat getrocknet, filtriert und eingeengt. Nach Reinigung durch Chromatographie (Kieselgel/$CH_2Cl_2$) und Umkristallisation aus Acetonitril werden 1,55 g 1,4-Di(2-fluor-6-octyloxypyridin-3-yl)benzol erhalten.

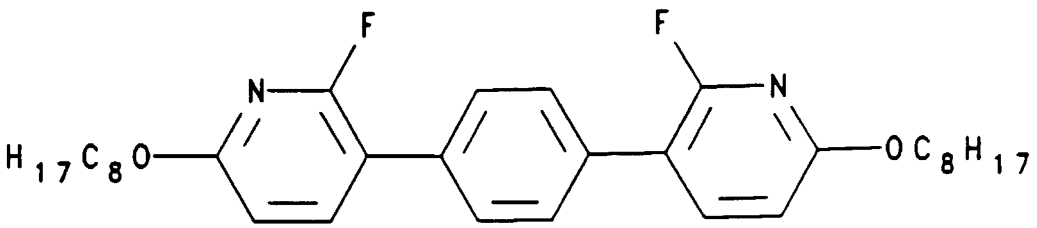

Die Verbindung zeigt die Phasenfolge:

X 91 $S_A$ 95 I

Referenz-Beispiel 17:

2,5 Di(2-fluor-6-octyloxypyridin-3-yl)pyrimidin

Die Darstellung erfolgt analog Beispiel 16.

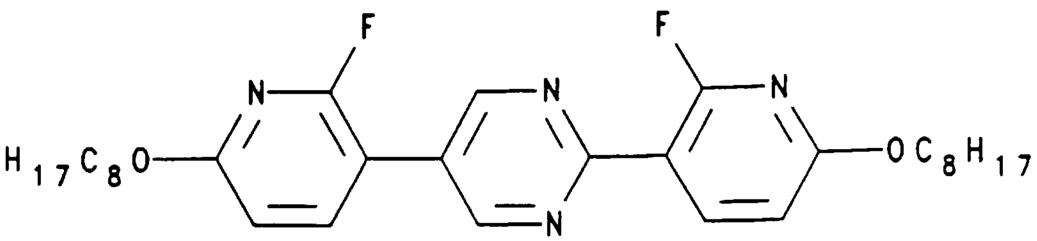

Die Verbindung zeigt die Phasenfolge:

X 91 $S_C$ 110 $S_A$ 116 I

Referenz-Beispiel 18:

2,5-Di(2-fluor-6-octyloxypyridin-3-yl)pyridin

Die Darstellung erfolgt analog Beispiel 16.

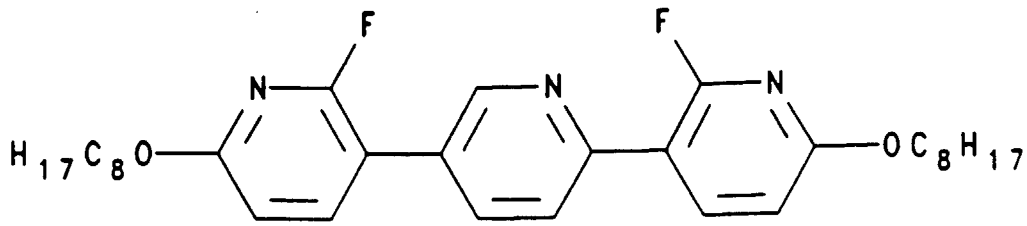

Die Verbindung zeigt die Phasenfolge:

X 65 $S_C$ 85 $S_A$ 104 I

Referenz-Beispiel 19:

3,6-Di(2-fluor-6-octyloxypyridin-3-yl)pyridazin

Die Darstellung erfolgt analog Beispiel 16.

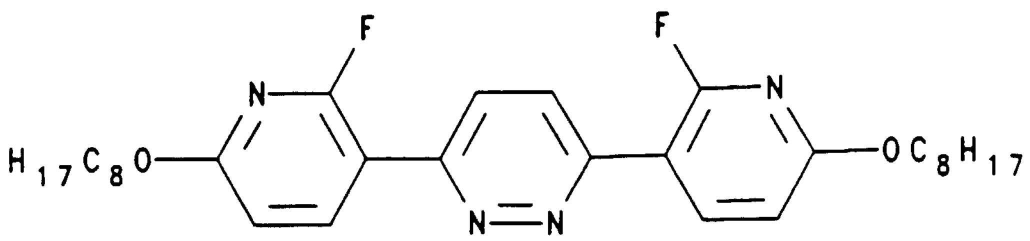

Die Verbindung zeigt die Phasenfolge:

X 100 $S_C$ 127 N 128 I

Beispiel 20:

trans-4-Pentylcyclohexancarbonsäure-[2-fluor-6-(4-octyloxyphenyl)pyridin-3-yl]ester

Darstellung nach Verfahren B:
0,29 g (0,91 mmol) 2-Fluor-3-hydroxy-6-(4-octyloxyphenyl)pyridin (hergestellt wie in Beispiel 3 beschrieben) 0,19 g (0,91 mmol) Dicyclohexylcarbodiimid, 0,16 g (0,91 mmol) trans-4-Pentylcyclohexancarbonsäure und 0,01 g 4-(N,N-Dimethylamino)pyridin werden in 10 ml Methylenchlorid 18 h bei Raumtemperatur gerührt. Nach Filtration, Einengen zur Trockne, chromatographischer Reinigung (Kieslgel/Hexan:Essigester (8:2)) und Umkristallisation aus Acetonitril werden 0,19 g trans-4-Pentylcyclohexancarbonsäure-[2-fluor-6-(4-octyloxyphenyl)pyridin-3-yl]ester erhalten.

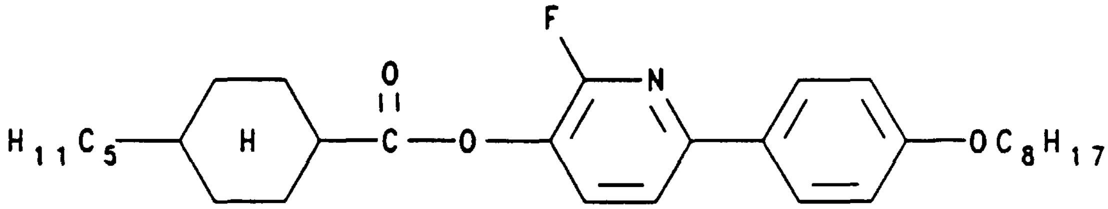

Die Verbindung zeigt folgende Phasenfolge:

X 62 $S_2$ 72 $S_C$ 137 N 184 I

Dieses Beispiel belegt, daß Verbindungen mit der erfindungsgemäßen Struktureinheit hervorragende flüssig-kristalline Eigenschaften, insbesondere eine breite smektische C und eine breite nematische Phase aufweisen.

Im Vergleich zur Pyrimidinverbindung

Phasenfolge: X 76 $S_C$ 115 $S_A$ 190 N 207 I

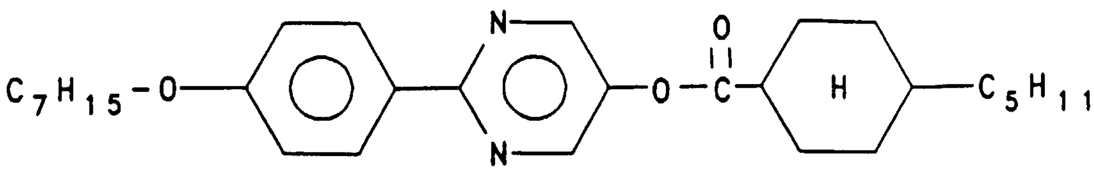

weist die erfindungsgemäße Verbindung einen um 14°C niedrigeren Schmelzpunkt, eine wesentlich breitere nematische Phase und eine breitere $S_C$-Phase auf.

Im Vergleich zur Fluor-pyrimidinverbindung

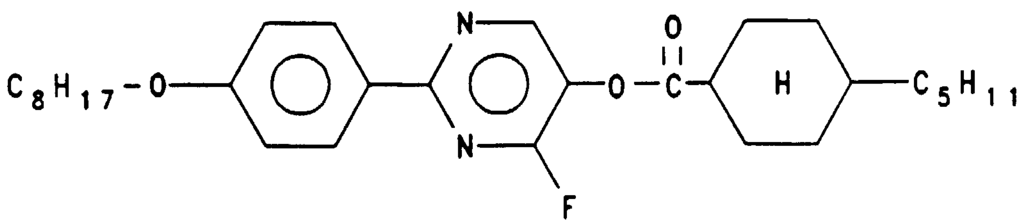

Phasenfolge: X 60 $S_C$ 67 N 188 I

weist die erfindungsgemäße Verbindung eine wesentlich breitere smektische C Phase auf.

Referenz-Beispiel 21:

trans-4-Pentylcyclohexancarbonsäure-[4-(2-fluor-3-octylpyridin-6-yl)phenyl)]ester

Darstellung nach Verfahren B:

Zu 35,24 g (130,0 mmol) tert.-Butyl-chlor-diphenylsilan und 11,25 g (65,0 mmol) 4-Bromphenol in 150 ml Dimethylformamid werden bei Raumtemperatur 11,06 g (162,5 mmol) Imidazol in 30 ml Dimethylformamid getropft. Nach einstündigem Rühren bei Raumtemperatur wird das Reaktionsgemisch auf 1000 ml 5 %ige

Natriumhydrogencarbonatlösung gegossen, zweimal mit 400 ml Dichlormethan extrahiert, die organische Phase mit Nathumchloridlösung gewaschen, über Natriumsulfat getrocknet, filtriert und zur Trockne eingeengt. Nach chromatrographischer Reinigung (Kieselgel/Hexan:Essigester = 8:2) werden 23,40 g 4-Bromphenyl-tert.-butyl-diphenylsilylether erhalten.

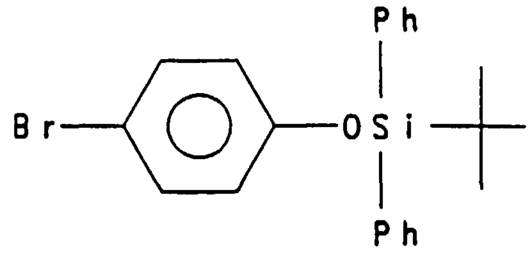

22,61 g (55,0 mmol) 4-Bromphenyl-tert.-butyl-diphenylsilylether in 40 ml Benzol und 35 ml (55,0 mmol) einer 1,6 molaren n-Butyllithiumlösung in Hexan werden unter Argon über Nacht bei Raumtemperatur gerührt und anschließend langsam bei 0°C unter Argon zu 4,50 ml (50,0 ml) 2,6-Difluorpyridin in 40 ml Tetrahydrofuran getropft. Nach dreistündiger Reaktionszeit bei 0°C wird mit Natriumchloridlösung versetzt, in Ether aufgenommen, die organische Phase mit Natriumchloridlösung gewaschen, über Natriumsulfat getrocknet, filtiert, vom Lösungsmittel befreit und chromatographisch (Kieselgel/Hexan:Essigester = 9:1) gereinigt. Es werden 6,80 g 6-(4-tert.-Butyldiphenylsilyloxyphenyl)-2-fluorpyridin erhalten.

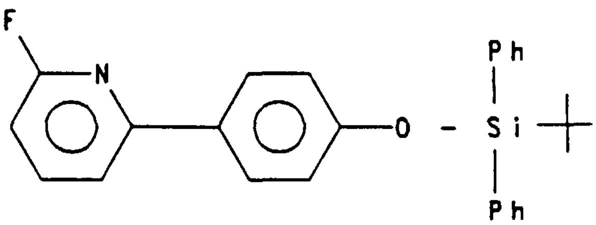

4,46 ml (31,8 mmol) Diisopropylamin in 55 ml Tetrahydrofuran werden bei 0°C 1 h mit 20 ml (31,8 mmol) einer 1,6 molaren n-Butyllithiumlösung in Hexan gerührt. Nach Abkühlen auf .78°C werden 6,80 g (15,9 mmol) 2-Fluor-6-(4-tert.-butyl-diphenylsilyloxyphenyl)pyridin in 500 ml Tetrahydrofuran zugetropft und 4 h bei - 78°C gerührt. Anschließend werden 11,9 g (63,25 mmol) Triisopropylborat in 30 ml Tetrahydrofuran bei -78°C zugetropft und das Reaktionsgemisch über Nacht gerührt, wobei es sich auf Raumtemperatur erwärmt. Nach Zugabe von 8 ml Essigsäure:Wasser = 4:1 und Rühren bei Raumtemperatur über Nacht wird mit Natriumchloridlösung versetzt, mit Ether extrahiert, die Etherphase mit Natriumchloridlösung gewaschen, über Natriumsulfat getrocknet, filtriert und eingeengt. Der Rückstand wird in 300 ml Tetrahydrofuran aufgenommen und mit 50 ml einer 17,5 %higen Wasserstoffperoxidlösung 4 h unter Rückfluß erhitzt. Nach Versetzen mit Natriumchloridlösung, Extraktion mit Ether, Waschen der organischen Phase mit Natriumsulfitlösung und Natriumchloridlösung, Trocknen über Natriumsulfat, Filtration, Einengen zur Trockne und chromatographischer Reinigung (Kieselgel/Hexan:Essigester = 8:2) werden 3,58 g 6-(4-tert.-Butyl-diphenylsilyloxyphenyl)-2-fluor-3-hydroxypyridin erhalten.

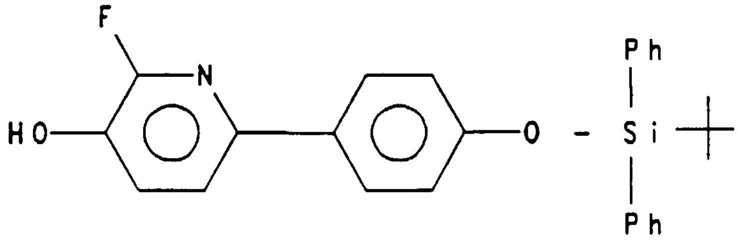

Zu 3,47 g (13,35 mmol) Triphenylphosphin in 100 ml Tetrahydrofuran werden bei 0°C 2,30 g (13,35 mmol) Azodicarbonsäurediethylester getropft und 30 min bei Raumtemperatur gerührt. Anschließend werden 3,58 g (8,10 mmol) 6-(4-tert.-Butyl-diphenylsilyloxyphenyl)-2-fluor-3-hydroxypyridin und 1,70 g (13,35 mmol) 1-Octanol zugegeben. Nach einer Reaktionszeit von 1,5 h bei Raumtemperatur wird das Lösungsmittel abdestilliert und der Rückstand chromatographisch (Kieselgel/Hexan:Essigester = 95:5) gereinigt, wobei 4,43 g 6-(4-tert.-Butyl-diphenylsilyloxyphenyl)-2-fluor-3-octyloxypyiridin erhalten werden.

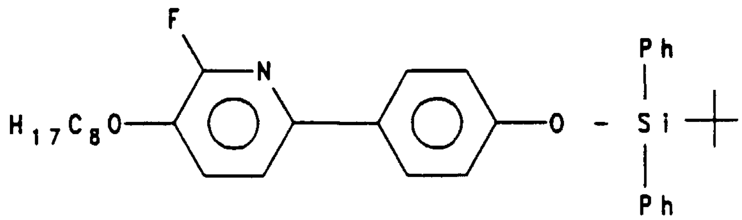

4,30 g (8,00 mmol) 6-(4-tert.-Butyl-diphenylsilyloxyphenyl)-2-fluor-3-octyloxypyridin werden in 16 ml einer 1-molaren Tetrabutylammoniumfluoridlösung in Tetrahydrofuran in 50 ml Tetrahydrofuran 2 h bei Raumtemperatur gerührt. Anschließend wird mit Natriumchloridlösung versetzt, mit Ether extrahiert, die Etherphase mit Natriumchloridlösung gewaschen, über Natriumsulfat getrocknet, zur Trockne eingeengt und chromatogaphisch (Kieselgel/Hexan:Essigester = 8:2) gereinigt. Es werden 2,16 g 2-fluor-6-(4-hydroxy-phenyl)-3-octyloxypyridin erhalten.

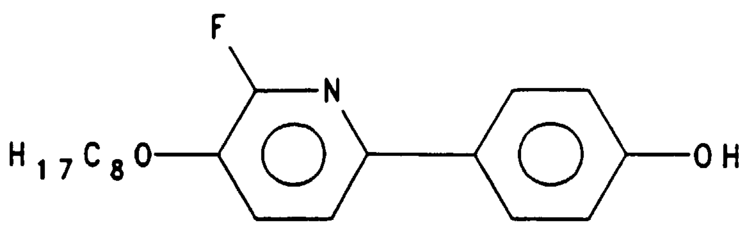

1,11 g (3,50 mmol) 2-Fluor-6-(4-hydroxyphenyl)-3-octyloxypyridin, 0,72 g (3,50 mmol) Dicyclohexylcarbodiimid, 0,69 g (3,50 mmol) trans-4-Pentylcyclohexancarbonsäue und 0,02 g 4-(N,N-Dimethylamino)pyridin werden in 20 ml Dichlormethan 3 h bei Raumtemperatur gerührt. Nach Filtration, Einengen zur Trockne, chromatographischer Reinigung (Kieselgel/Hexan:Essigester = 8:2) und Umkristallisation aus n-Hexan werden 1,00 g trans-4-Pentylcyclohexancarbonsäure-[4-(2-fluor-3-octylpyridin-6-yl)phenyl)]ester erhalten.

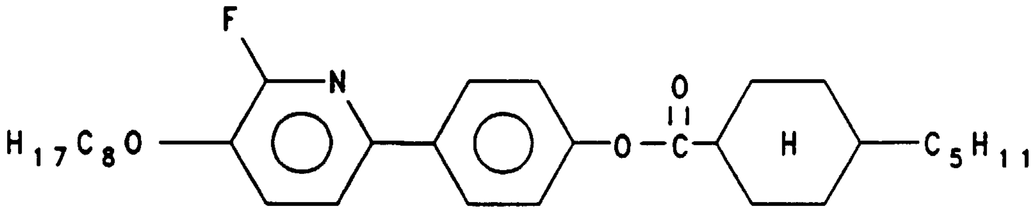

Die Verbindung zeigt folgende Phasenfolge:

X 75 $S_X$ 91 $S_C$ 162 N 188 ← I

Referenz-Beispiel 22:

2-Fluor-6-(4-octyloxyphenyl)-3-trans-4-pentylcyclohexylmethoxy)pyridin

Darstellung nach Verfahren B:
Analog Beispiel 3 werden aus 1,85 g (7,05 mmol) Triphenylphosphin, 1,23 g (7,05 mmol) Azodicarbonsäurediethylester, 1,5 g (4,70 mmol) 2-Fluor-3-hydroxy-6-(4-octyloxyphenyl)pyridin (hergestellt wie in Beispiel 3 beschrieben) und 0,86 g (4,70 mmol) trans-4-Pentylcyclohexylmethanol in 30 ml Tetrahydrofuran 0,87 g 2-Fluor-6-(4-octyloxyphenyl)-3-(trans-4-pentylcyclohexylmethoxy)pyridin erhalten. Die Verbindung zeigt die Phasenfolge:

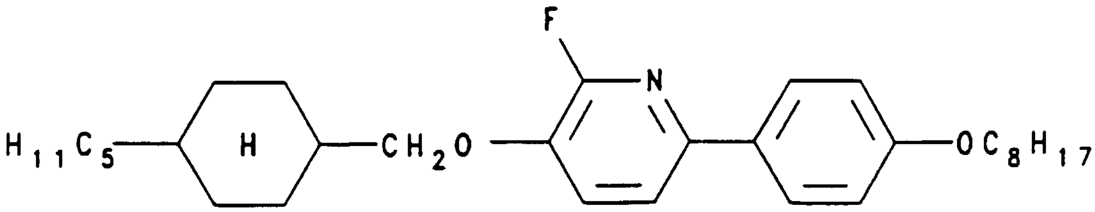

X 79 $S_C$ 150 $S_A$ 155 N 158 I

Referenz Beispiel 23:

2-Fluor-3-octyloxy-6-[trans-4-pentylcyclohexyl] pyridin

Darstellung nach Verfahren B:
12,48 g (40,40 mmol) 4-(Trans-4-pentylcyclohexyl)brombenzol in 30 ml Benzol werden mit 25,20 ml (40,40 mmol) einer 1,6 molaren n-Butyllithiumlösung in Hexan unter Argon 18 h bei Raumtemperatur gerührt. Danach wird das Reaktionsgemisch langsam bei O°C unter Argon zu einer Lösung von 4,20 g (36,70 mmol) 2,6-Difluorpyridin in 30 ml Tetrahydrofuran getropft. Nach einstündiger Reaktionszeit bei O°C wird zwischen Ether und Natriumchloridlösung verteilt, die organische Phase mit Natriumchloridlösung gewaschen, über Natriumsulfat getrocknet, filtriert und vom Lösungsmittel befreit. Nach chromatographischer Reinigung werden 0,94 g 2-Fluor-6-[4-(trans-4-pentylcyclohexyl)phenyl]pyridin erhalten.

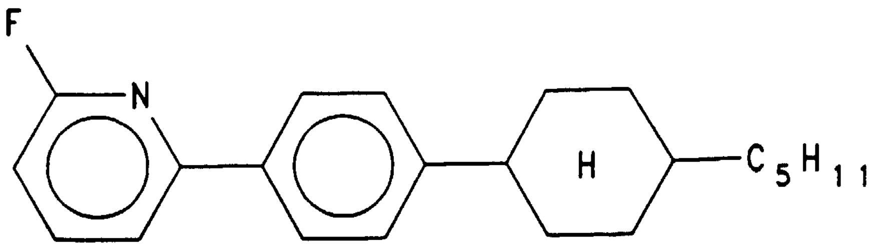

0,49 ml (3,5o mmol)Diisopropylamin in 10 ml Tetrahydrofuran werden bei 0°C eine Stunde mit 2,20 ml (3,50 mmol) einer 1,6 molaren n-Butyllithiumlösung in Hexan gerührt. Nach Abkühlen auf -78°C werden 0,94 g (2,90 mmol) 2-Fluor-6-[4-(trans-4-pentylcyclohexyl)phenyl]pyridin in 120 ml Tetrahydrofuran zugetropft und 4 h bei - 78°C gerührt. Anschließend werden 1,31 g (6,96 mmol) Triisopropylborat bei -78°C zugetropft und das Reaktionsgemisch über Nacht gerührt, wobei es sich auf Raumtemperatur erwärmt. Nach Zugabe von 4,40 ml 10%iger Salzsäure und einstündigem Rühren bei Raumtemperatur wird mit Natriumchloridlösung versetzt, mit Ether extrahiert, die Etherphase mit Natriumchloridlösung gewaschen, über Natriumsulfat getrocknet, filtriert und eingeengt. Der Rückstand wird in 50 ml Tetrahydrofuran aufgenommen und mit 8,80 ml einer 17,5%igen Wasserstoffperoxidlösung 4 h unter Rückfluß erhitzt. Nach Versetzen mit Natriumchloridlösung, Extraktion mit Ether, Waschen der organischen Phase mit Natriumsulfitlösung und Natriumchloridlösung, Trocknen über Natriumsulfat, Filtration, Einengen zur Trockne und chromatographischer Reinigung (Kieselgel/Hexan:Essigester = 9:1) werden 0,31 g 2-Fluor-3-hydroxy-6-[4-(trans-4-pentylcyclohexyl)phenyl]pyridin erhalten.

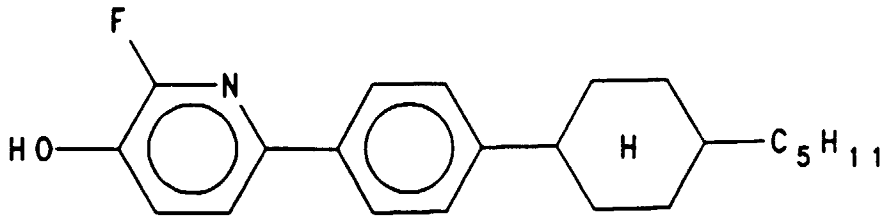

Zu 0,39 g (1,50 mmol) Triphenylphosphin in 10 ml Tetrahydrofuran werden bei O°C 0,26 g (1 ,50 mmol) Azodicarbonsäurediethylester getropft und 30 min bei Raumtemperatur gerührt. Anschließend werden 0,31 g (0,91 mmol) 2-Fluor-3-hydroxy-6-[4-trans-4-pentylcyclohexyl)phenyl]pyridin und 0,19 g (1,50 mmol) 1-Octanol zugegeben. Nach einer Reaktionszeit von 2 h bei Raumtemperatur wird das Lösungsmittel abdestilliert und der Rückstand chromatographisch (Kieselgel/Hexan:Essigester = 9:1) und durch Umkristallisation aus Acetonitril gereinigt. Es werden 0,20 g 2-Fluor-3-octyloxy-6-[4-(trans-4-pentylcyclohexyl)phenyl]pyridin erhalten.

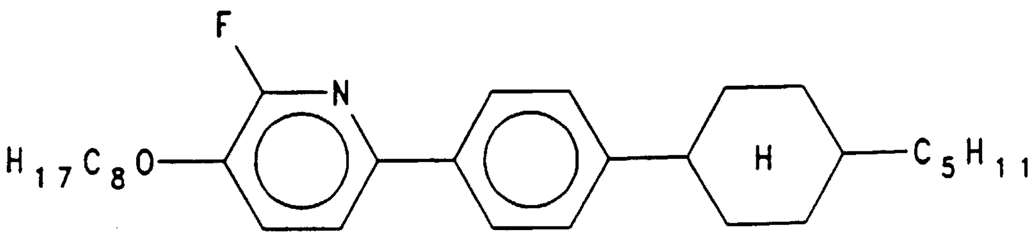

Die Verbindung zeigt folgende Phasenfolge:

X 77 $S_C$ 137 $S_A$ 150 N 161 I

Verbindungen dieses Typs sind besonders vorteilhaft, da sie neben einem niedrigen Schmelzpunkt alle für ferroelektrische Mischungen notwendigen Phasen aufweisen.

Referenz- Beispiel 24:

2-Fluor-3-hexyloxy-6-[4-(trans-4-pentylcyclohexyl)phenyl]pyridin

Die Darstellung erfolgt analog Beispiel 23.

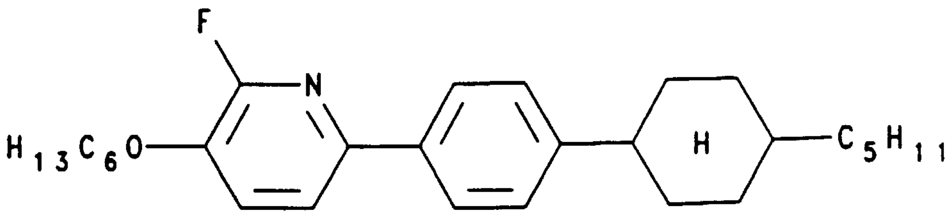

Die Verbindung zeigt die Phasenfolge:

X 80 $S_3$ 84 $S_C$ 137 $S_A$ 148 N 168 I

Referenz-Beispiel 25:

3-Decyloxy-2-fluor-6-[4-(trans-4-pentylcyclohexyl)phenyl]pyridin

Die Darstellung erfolgt analog Beispiel 23.

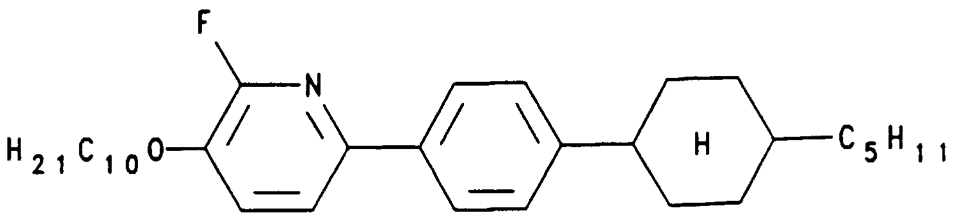

Die Verbindung zeigt die Phasenfolge:

X 76 $S_C$ 133 $S_A$ 151 N 156 I

Referenz-Beispiel 26:

3-Dodecyloxy-2-fluor-6-[4-(trans-4-pentylcyclohexyl)phenyl]pyridin

Die Darstellung erfolgt analog Beispiel 23.

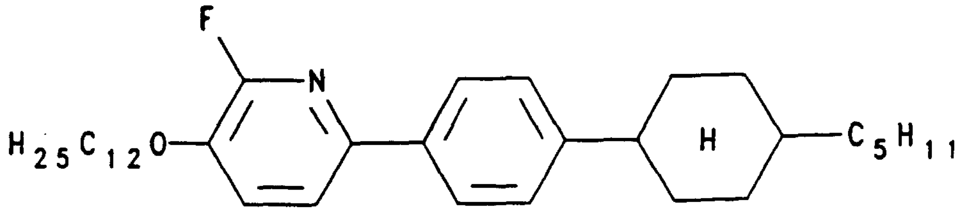

Die Verbindung zeigt die Phasenfolge:

X 80 $S_C$ 127 $S_A$ 148 N 150 I

Beispiel 27:

3-[4-(Butyldimethylsilyl)butyloxy]-2-fluor-6-[4-(trans-4-pentylcyclohexyl)phenyl]pyridin

Die Darstellung erfolgt analog Beispiel 23.

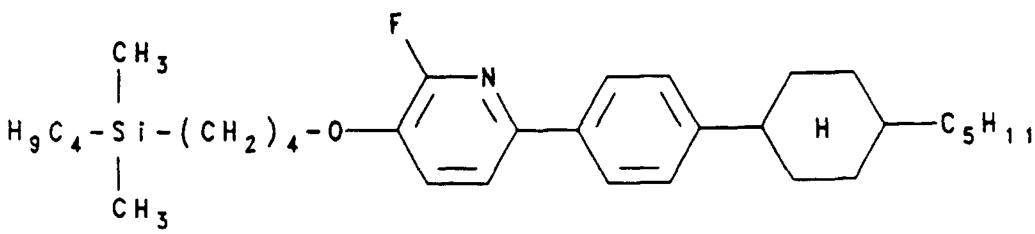

Die Verbindung zeigt die Phasenfolge:

X 61 $S_C$ 91 $S_A$ 102 I

Referenz-Beispiel 28:

2-Fluor-6-octyloxy-3-[4-(trans-4-pentylcyclohexyl)-phenyl]pyridin

Darstellung nach Verfahren B:
Analog Beispiel 5 werden aus 2,27 g (7,4 mmol) 1-Brom-4-(trans-4-pentylcyclohexyl)benzol, 2,00 g (7,4 mmol) 2-Fluor-6-octyloxypyridin-3-boronsäure, 0,28 g (0,23 mmol) Tetrakistriphenylphosphinpalladium [O] und 2,5 g (23,1 mmol) Natriumcarbonat in 50 ml Benzol, 35 ml Ethanol und 1o ml Wasser 2,0 g 2-Fluor-6-octyloxy-3-[4-trans-4-pentylcyclohexyl)-phenyl]pyridin erhalten.

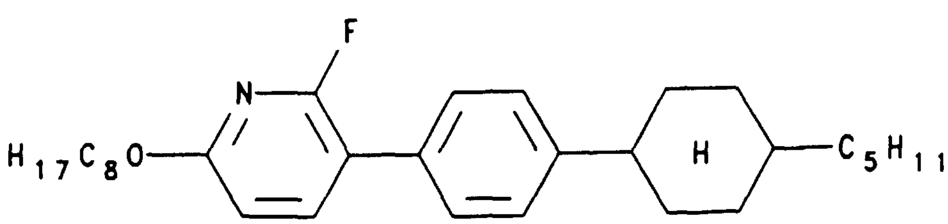

Die Verbindung zeigt die Phasenfolge:

X 78 $S_A$ 110 N 117 I

Referenz-Beispiel: 29

6-Decyloxy-2-fluor-3-[4-(trans-4-pentylcyclohexyl)phenyl]pyridin

Die Darstellung erfolgt analog Beispiel 28.

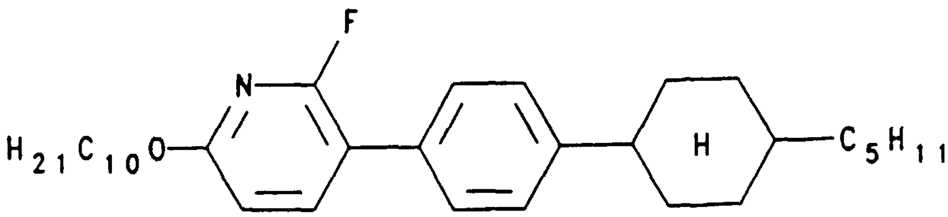

Die Verbindung zeigt die Phasenfolge:

X 64 $S_A$ 108 N 112 I

Referenz-Beispiel 30:

6-Dodecyloxy-2-fluor-3-[4-trans-4-pentylcyclohexyl)phenyl]pyridin

Die Darstellung erfolgt analog Beispiel 28.

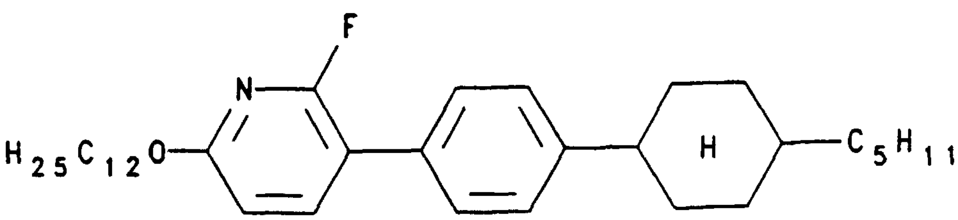

Die Verbindung zeigt die Phasenfolge:

X 62 $S_A$ 104 N 107 I

Beispiel 31:

2-Fluor-3-[4-(4-octylphenyl)phenyl]-6-(4-octyloxyphenyl)pyridin

Die Darstellung erfolgt analog Beispiel 10.

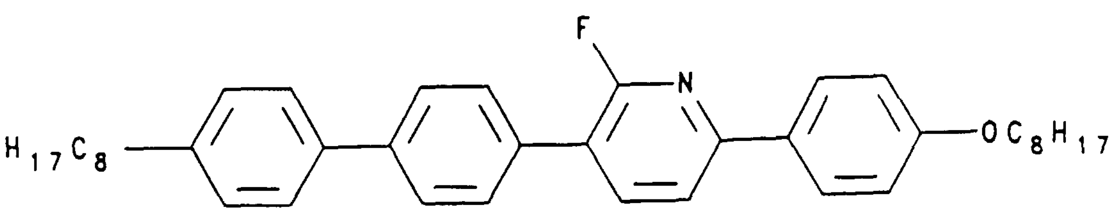

Die Verbindung zeigt die Phasenfolge:

X 73 $S_X$ 173 $S_C$ 273 $S_A$ 275 N 281 I

Beispiel 32:

2-Fluor-3-(5-octyl-1,3-dioxaborinan-2-yl)-6-(4-octyloxyphenyl)pyridin

Darstellung nach Verfahren B:
1,00 g (3,00 mmol) 2-Fluor-6-(4-octyloxyphenyl)pyridin-3-boronsäure (hergestellt wie in Beispiel 10 beschrieben), 0,56 g (3,00 mmol) 2-Octylpropan-1,3-diol und 1,50 g Natriumsulfat in 30 ml Tetrahydrofuran werden 48 h bei Raumtemperatur gerührt. Anschließend wird vom Natriumsulfat abfiltriert, eingeengt und aus Hexan umkristallisiert. Es werden 1,24 g 2-Fluor-3-(5-octyl-1,3-dioxaborinan-2yl)-6-(4-octyloxyphenyl)pyridin erhalten.

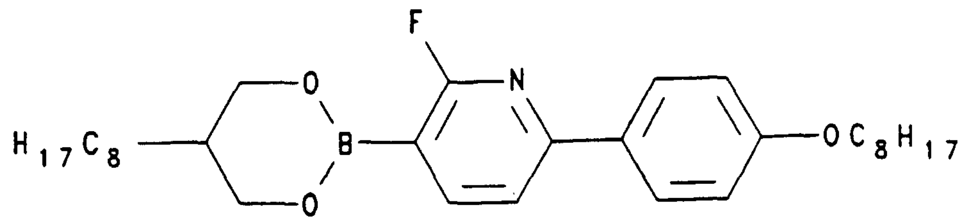

Die Verbindung zeigt die Phasenfolge:

X 91 $S_C$ 127 N 142 I

Beispiel 33:

2-Fluor-3-(5-octyl-1,3-dioxan-2-yl)-6-(4-octyloxyphenyl)pyridin

Darstellung nach Verfahren B:
2,55 ml (18,2 mmol) Diisopropylamin in 30 ml Tetrahydrofuran werden bei 0°C eine Stunde mit 11,4 ml (18,2 mmol) einer 1,6 molaren n-Butyllithiumlösung in Hexan gerührt. Nach Zugabe von 170 ml Tetrahydrofuran und Abkühlen auf -78°C werden 3,64 g (12,1 mmol) 2-Fluor-6-(4-octyloxyphenyl)pyridin (hergestellt wie in Beispiel 2 beschrieben) in 90 ml Tetrahydrofuran zugetropftund 4 h bei -78°C gerührt. Anschließend werden 1,86 ml (24,2 mmol) N,N.Dimethylformamid bei -78°C zugetropft und das Reaktionsgemisch über Nacht gerührt, wobei es sich auf Raumtemperatur erwärmt. Zur Aufarbeitung wird auf Natriumchloridlösung gegossen, mit Ether extrahiert, die organische Phase mit Wasser gewaschen, über Natriumsulfat getrocknet, filtriert und eingeengt. Nach chromatographischer Reinigung (Kieselgel/$CH_2Cl_2$) werden 2,20 g 2- Fluor-3-formyl-6-(4-octyloxyphenyl)pyridin erhalten.

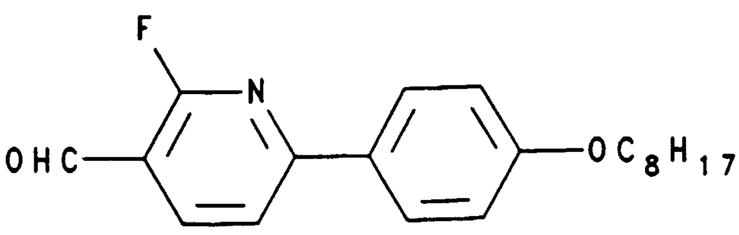

2,20 g (6,68 mmol) 2-Fluor-3-formyl-6-(4-octyloxy-phenyl)pyridin, 1,26 g (6,68 mmol) 2-Octylopropan-1,3-diol, 0,02 g (0,10 mmol) p-Toluolsulfonsäure und 3,00 g Natriumsulfat werden in 100 ml Toluol 68 h bei Raumtemperatur gerührt. Anschließend werden 0,14 ml (1,00 mmol) Triethylamin zugegeben, vom Natriumsulfat abfiltriert, eingeengt und chromatographisch (Kieselgel/Hexan:Essigester =9:1) und durch Umkristallisation aus Acetonitril gereinigt. Es werden 1,40 g 2 Fluor-3-(5-octyl-1,3-dioxan-2-yl)-6-(4-octyloxyphenyl)pyridin erhalten.

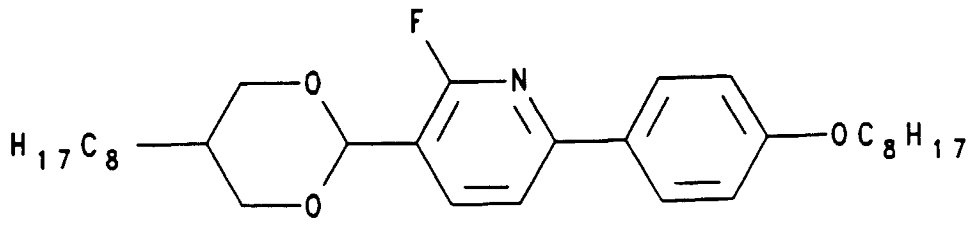

Die Verbindung zeigt die Phasenfolge:

X 86 $S_C$ 103 N 146 I

Referenz-Beispiel 34:

2-Fluor-6-octyloxy-3-(6octyloxynaphthalin-2-yl)-pyridin

Darstellung nach Verfahren B:
Analog Beispiel 5 werden aus 2,48 g (7,4 mmol) 2-Fluor-6-octyloxypyridin-3-boronsäure, 0,28 g (0,23 mmol) Tetrakistriphenylphosphinpalladium [0] und 2,5 g (23,1 mmol) Natriumcarbonat in 50 ml Benzol, 35 ml Ethanol und 10 ml Wasser 2,00 g 2-Fluor-6-octyloxy-3-(6-octyloxynaphthalin-2-yl)pyridin erhalten.

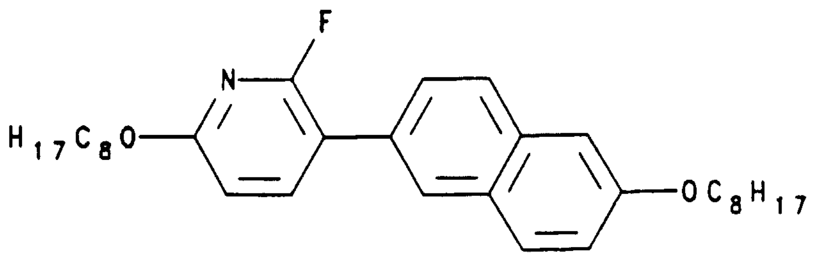

Die Verbindung zeigt die Phasenfolge

X 58 $S_A$ 86 I

Referenz-Beispiel 35:

2-Fluor-3-octyloxy-6-(6-octyloxynaphthalin-2-yl)pyridin

Darstellung nach Verfahren B:
Aus 2,4 g (99,0 mmol) Magnesium und 30 g (89,6 mmol) 2-Brom-6-octyloxynapththalin in 250 ml Tetrahydrofuran werden in 3 h bei 55° C die Lösung der Grignardverbindung hergestellt, welche zu einer auf -78° C abgekühlten Lösung von 41,36 ml (179,2 mmol) Triisopropylborat in 100 ml Tetrahydrofuran getropft und über Nacht gerührt wird. Anschließend werden 130 ml 10 %ige Salzsäure zugetropft und 1 h bei Raumtemperatur gerührt, zwischen Natriumchloridlösung und Ether verteilt, die organische Phase mit Natriumchloridlösung gewaschen, über Natriumsulfat getrocknet, eingeengt und aus Hexan umkristallisiert, wonach 14,5 g 6-Octyloxynaphthalin-2-boronsäure erhalten werden.

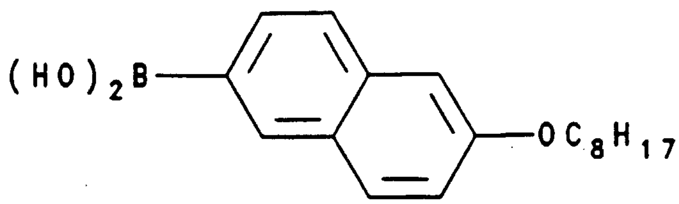

100 g (422,10 mmol) 2,6-Dibrompyrldin, 36,79 g (633,15 mmol) Kaliumfluorid und 11,16 g 18-Krone-6 werden in einer Destillationsapparatur unter Vakuum (200 mbar) auf 190° C erhitzt und das Reaktionsprodukt abdestilliert. Nach erneuter destillativer Reinigung werden 43,90 g 2-Brom-6-fluorpyridin mit einem Siedepunkt von 70° C bei 15 mbar erhalten.

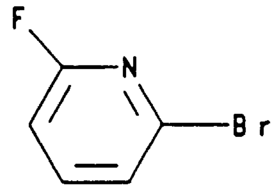

8,51 g (50 mmol) 2-Brom-6-fluorpyridin, 14,5 g (50 mmol) 6-Octyloxynaphthalin-2-boronsäure, 1,73 g (1,5 mmol) Tetrakistriphenylphosphinpalladium [O] und 10,6 g (100 mmol) Natriumcarbonat werden in 375 ml Benzol, 250 ml Ethanol und 125 ml Wasser 3 h unter Rückfluß erhitzt. Anschließend wird zwischen Natriumchloridlösung und Ether verteilt, die organische Phase mit Natriumchloridlösung gewaschen, über Natriumsulfat getrocknet, eingeengt und chromatographisch (Kieselgel/Hexan:Essigester = 9:1) gereinigt. Es werden 12,08 g 2-Fluor-6-(6-octyloxynaphthalin-2-yl)pyridin erhalten.

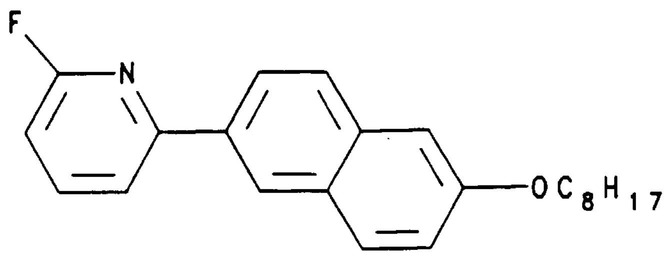

1,08 g (10,68 mmol) Diisopropylamin in 20 ml Tetrahydrofuran werden bei 0° C 1 h mit 6,7 mol (10,68 mmol) einer 1,6 molaren n-Butyllithiumlösung in Hexan gerührt.

Nach Abkühlen auf -78° C werden 2,50 g (7,12 mmol) 2-Fluor-6(6-octyloxynaphthalin-2-yl)pyridin in 250 ml Tetrahydrofuran zugetropft und 4 h bei -78° C gerührt. Anschließend werden 3,3 ml (14,24 mmol) Triisopropylborat bei - 78° C zugetropft und das Reaktionsgemisch über Nacht gerührt, wobei es sich auf Raumtemperatur erwärmt. Nach Zugabe von 50 ml konzentrierter Ammoniumchloridlösung und 1 h Rühren bei Raumtemperatur wird mit Natriumchloridlösung versetzt, mit Ether extrahiert, die Etherphase mit Natriumchloridlösung gewaschen, über Natriumsulfat getrocknet, filtriert und eingeengt. Der Rückstand wird in 70 ml Tetrahydrofuran aufgenommen und mit 25 ml einer 17,5 %igen Wasserstoffperoxidlösung 2 h unter Rückfluß erhitzt. Nach Versetzen mit Natriumchloridlösung, Extraktion mit Ether, Waschen der organischen Phase mit Natriumsulfitlösung und Natriumchloridlösung, Trocknen über Natriumsulfat, Filtration, Einengen zur Trockne und chromatographischer Reinigung (Kieselgel/Hexan:Essigester = 7:3) werden 1,55 g 2-Fluor-3-hydroxy-6-(6-octyloxynaphthalin-2-yl)pyridin erhalten.

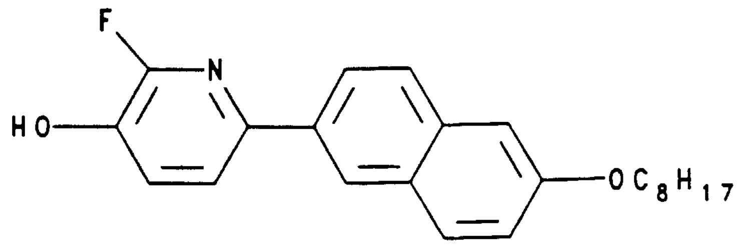

Zu 1 ,60 g (6,11 mmol) Triphenylphosphin in 50 ml Tetrahydrofuran werden bei 0° C 1,06 g (6,11 mmol) Azodicarbonsäurediethylester getropft und 0,5 h bei Raumtemperatur gerührt. Anschließend werden 1,50 g (4,08 mmol) 2-Fluor-3-hydroxy-6-(6-octyloxy-naphthalin-2-yl)pyridin und 0,80 g (6,11 mmol) 1-Octanol zugegeben. Nach einer Reaktionszeit von 1 h bei Raumtemperatur wird das Lösungsmittel abdestilliert und der Rückstand chromatographish (Kieselgel/Hexan:EE = 9:1) und durch Umkristallisation aus Acetonitril gereinigt, wobei 1,74 g 2-Fluor-3-octyloxy-6-(6-octyloxynaphthalin-2-yl)pyridin erhalten werden.

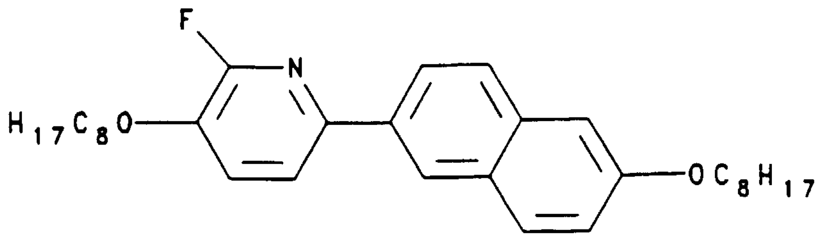

Die Verbindung zeigt die Phasenfolge:

X 105 $S_2$ 95 $S_C$ 136 I

Beispiel 36:

[(2S,3S)-3-Pentyloxiran-2-yl]methyl-[2-fluor-6-(4-octyloxyphenyl)pyridin-3-yl]ether

Darstellung nach Verfahren B:
Zu 0,67 g (2,55 mmol) Triphenylphosphin in 15 ml Tetrahydrofuran werden bei 0° C 0,44 g (2,55 mmol) Azodicarbonsäurediethylester getropft und 30 min bei Raumtemperatur gerührt. Anschließend werden 0,54 g (1,70 mmol) 2-Fluor-3-hydroxy-6-(4-octyloxyphenyl)pyridin (hergestellt nach Beispiel 3) und 0,25 g (1,70 mmol) 2-[(2S,3S)-3-Pentyloxiranyl]methanol zugegeben. Nach einer Reaktionszeit von 18 h bei Raumtemperatur wird das Lösungsmittel abdestilliert und der Rückstand chromatographisch (Kieselgel/Hexan:Essigester = 8:2) gereinigt. Die Umkristallisation aus Hexan:Essigester (8:2) ergibt 0,28 g [(2S,3S)-3-Pentyloxiran-2-yl]methyl-[2-fluor-6-(4-octyloxyphenyl)pyridin-3-yl]ether mit [$\alpha$ $^{20}_{D}$ (2,4 % in $CH_2Cl_2$) = -15,75°.

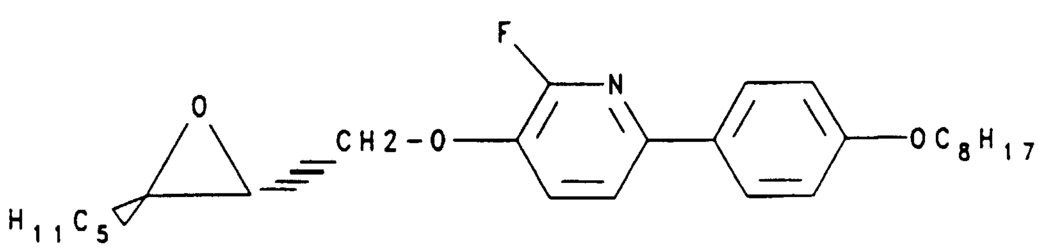

Die Verbindung zeigt folgende Phasenfolge:

X 86 $S_C$ 99 I

Beispiel 37:

[(2S,3S-3-Pentyloxiran-2-yl]methyl-[4-(2-fluor-3-octyloxypyridin-6-yl)phenyl]ether

Darstellung nach Verfahren B:
Zu 1,37 g (5,25 mmol) Triphenylphosphin in 20 ml Tetrahydrofuran werden bei 0° C 0,91 g (5,25 mmol) Azodicarbonsäurediethylester getropft und 30 min bei 0° C gerührt. Anschließend werden 1,11 g (3,50 mmol) 2-Fluor-6-(4-hydroxyphenyl)-3-octyloxypyridin (hergestellt wie in Beispiel 21 beschrieben) und 0,75 g (5,25 mmol) 2-[(2S,3S)-3-Pentyloxiran-2-yl]methanol zugegeben. Nach einer Reaktionszeit von 18 h bei Raumtemperatur wird das Losungsmittel abdestilliert und der Rückstand chromatographisch (Kieselgel/Hexan:Essigester = 8:2) gereinigt. Die Umkristallisation aus Hexan ergibt 0,85 g [(2S,3S-3-Pentyloxiran-2-yl]methyl-[4-(2-fluor-3-octyloxypyridin-6-yl)phenyl]ether mit [$\alpha{}^{20}_{D}$ (2,6 % in $CH_2Cl_2$) = 18,01°

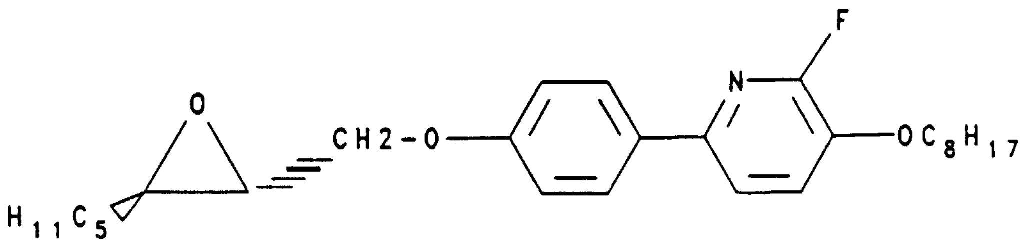

Die Verbindung zeigt die Phasenfolge:

X 93 $S_C$ 113 I

Beispiel 38:

[(2S,3S)-3-Pentyloxiran-2-yl]methyl-[2-fluor-6-(4-(trans-4-pentylcyclohexyl)phenyl)pyridin-3-yl]-ether

Die Darstellung erfolgt analog Beispiel 37:

[$\alpha{}^{20}_{D}$ (2,3 % in $CH_2Cl$) = -12,15

Die Verbindung zeigt die Phasenfolge:

X 103 $S_C$ 132 $S_A$ 151 N 163 I

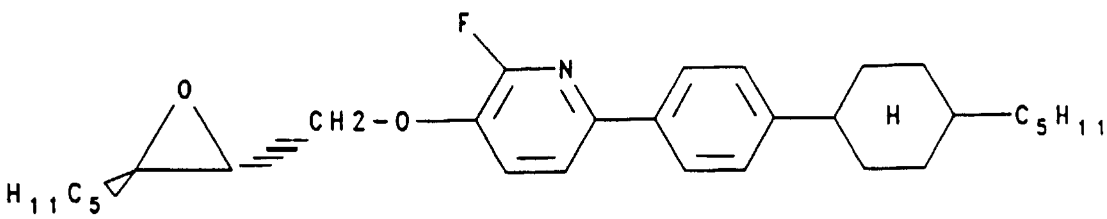

Anwendungsbeispiel 1:

a) Eine ferroelektrische Mischung, die aus den Komponenten

| | |
|---|---|
| 5-Octyloxy-2-(4-hexyloxyphenyl)pyrimidin | 22,8 Mol-% |
| 5-Octyloxy-2-(4-butyloxyphenyl)pyrimidin | 24,0 Mol-% |
| 5-Octyloxy-2-(4-decylocyphenyl)pyrimidin | 19,2 Mol-% |
| 5-Octyloxy-2-(4-octyloxyphenyl)pyrimidin | 10,5 Mol-% |
| trans-4-Pentyl-cyclohexancarbonsäure-(4'-(5-decylpyrimidin-2-yl))phenylester | 13,5 Mol-% |
| ((2S,3S)-3-Pentyloxiran-2-yl)methyl-(2-fluor-6-(4-octylocyphenyl)pyridin-3-yl)ether | 10,0 Mol-% |

besteht, zeigt folgende flüssigkristallinen Phasenbereiche:

$S^*_C$ 87 N 101 I

Sie weist bei einer Temperatur von 20° C eine spontane Polarisation von 9,5 nC/cm$^2$ auf und schaltet bei einer Feldstärke von 10 V/µm mit einer Schaltzeit von 350 µs.

b) Im Vergleich dazu weist eine bekannte flüssigkristalline Mischung (DE 38 31 226.3), die sich von der obengenannten Mischung nur dadurch unterscheidet, daß sie keinen Dotierstoff enthält, folgende Phasenbereiche auf.

X 95 $S_C$ 84 $S_A$ 93 N 105 I

Diese Mischung belegt, daß mit den erfindungsgemäßen Verbindungen schnell schaltende ferroelektrische Mischungen herstellbar sind.

Anwendungsbeispiel 2:

a) Eine Mischung, die aus den Komponenten

| | |
|---|---|
| 5-Octyloxy-2-(4-hexyloxyphenyl)pyrimidin | 16,4 Mol-% |
| 5-Octyloxy-2-(4-decyloxyphenyl)pyrimidin | 10,9 Mol-% |
| 5-Decyl-2-(4-hexyloxyphenyl)pyrimidin | 10,6 Mol-% |
| 5-Octyl-2-(4'-(7''-cyclopropylheptyloxy)-carbonyloxy-phenyl)-pyrimidin | 11,0 Mol-% |
| 5-(8'''-Cyclopropyloctyloxy)-2-(4''-trans-pentyl-cylcohexyl-4'phenyl)-pyrimidin | 12,7 Mol-% |
| trans-4-Pentyl-cyclohexancarbonsäure 4'-(8''-cyclopropyloctyl)-pyrimidin-2-yl-phenylester | 7,8 Mol-% |
| 5-(5''-Cyclopropylpentyloxy-2-(4'-hexyloxyphenyl)-pyrimidin | 11,7 Mol-% |
| ((2S,3S)-3-Pentyloxiran-2-yl)methyl-(4-(2-fluor-3-octyoxypyridin-6-yl)phenyl)ether | 10 Mol-% |

besteht, zeigt folgende flüssigkristalline Phasenbereiche:

$S^*_C$ 70 N* 84 I

Sie weist bei einer Temperatur von 25° C eine spontane Polarisation von 2,7 nC/cm$^2$ auf.

b) Im Vergleich dazu weist die flüssigkristalline Mischung, die sich von der oben genannten Mischung nur dadurch unterscheidet, daß sie keinen Dotierstoff enthält, folgende Phasenbereiche auf.

X -13 $S_C$ 65 $S_A$ 70 N 86 I

Die Anwendungsbeispiele 1 und 2 belegen zudem, daß die Zugabe der erfindungsgemäßen Verbindungen zu einer Erhöhung des $S_C$-Phasenbereiches führt.

Anwendungsbeispiel 3

Eine Mischung besteht aus

30 Mol-% trans-4-Pentyl-cyclohexancarbonsäure-[2-fluor-6-(4-octyloxyphenyl)pyridin-3-yl]ester
Komponente A

70 Mol-% 4-Ethyl-2-fluoro-4-[2-(trans-4-n-pentylcyclohexyl)-ethyl]-biphenyl
Komponente B

und weist einen Klärpunkt von 131°C auf und kristallisiert bei -23°C.

Im Vergleich dazu weist die Komponente B folgende Phasenumwandlungen auf:

X 24 $S_8$ (13) N 103,4 I

Die Zugabe der erfindungsgemäßen Komponente führt zu einer Erniedrigung des Kristallisationspunktes und zu einer Erhöhung des nematischen Phasenbereichs. Dieses Beispiel belegt, daß die erfindungsgemäßen Komponenten besonders gut geeignet sind, den nematischem Bereich von Flüssigkristallen zu verbreitern. Hervorzuheben ist ferner, daß die Verbindung in diesem Beispiel die dielektrische Anisotropie der Mischung verkleinert.

**Patentansprüche**

**1.** Fluorpyridinverbindung der allgemeinen Formel I

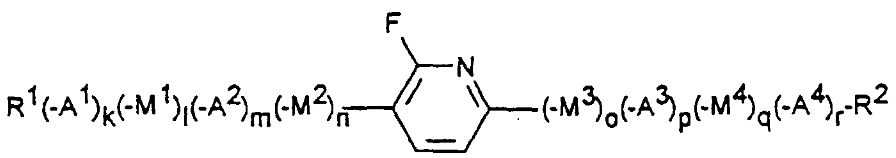

in der die Symbole folgende Bedeutung haben

$R^1$, $R^2$ unabhängig voneinander H, F, CN oder geradkettiges oder verzweigtes (mit oder ohne asymmetrisches C-Atom) Alkyl mit 1 bis 16 C-Atomen, wobei auch eine -$CH_2$-Gruppe durch -O-, -S-, -CO-, -CO-O-, -O-CO-, -O-CO-O-, -CH=CH-, -C≡C-, Δ oder -Si$(CH_3)_2$- ersetzt sein kann, oder eine der nachfolgenden chiralen Gruppen:

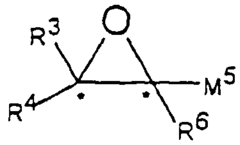 , 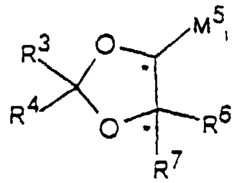 , 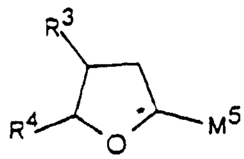 , 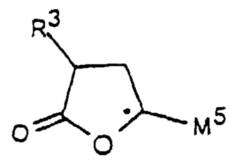 ,

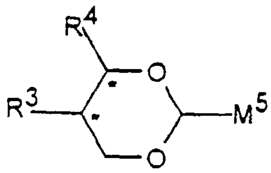 ,

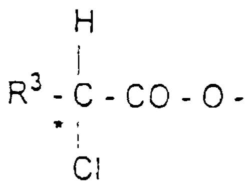 , 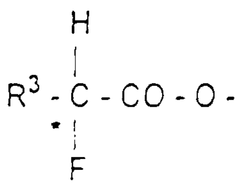 , 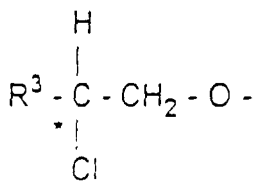 , 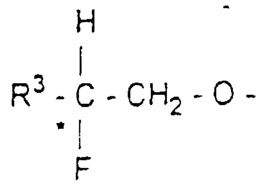 ,

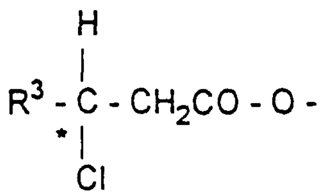 , 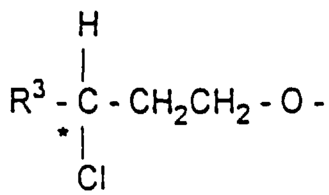 ,

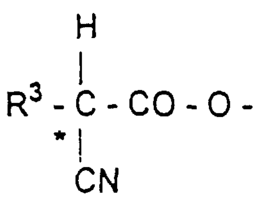 , 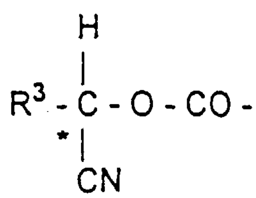

$A^1$, $A^2$, $A^3$, $A^4$ gleich oder verschieden 1,4-Phenylen, Pyrazin-2,5-diyl, Pyridazin-3,6-diyl, Pyridin-2,5-diyl, Pyrimidin-2,5-diyl, wobei ein oder zwei H-Atome durch F ersetzt sein können, trans-1,4-Cyclohexylen, (1,3,4)-Thiadiazol-2,5-diyl, 1,3-Dioxan-2,5-diyl, Naphthalin-2,6-diyl, Bicyclo[2.2.2.]octan-1,4-diyl oder 1,3-Dioxaborinan-2,5-diyl;

$M^1$, $M^2$, $M^3$, $M^4$ gleich oder verschieden -O-, -CO-, -CO-O-, -O-CO-, $-CH_2-O-$, $-O-CH_2-$,$-CH_2-CH_2$,, -CH=CH- oder -C≡C-;

$R^3$, $R^4$, $R^6$, $R^7$ unabhängig voneinander H oder geradkettiges oder verzweigtes Alkyl mit 1 bis 10 C-Atomen oder $R^3$ und $R^4$ zusammen auch $-(CH_2)_4-$ oder $-(CH_2)_5-$, wenn als Substituenten an ein Dioxolan System gebunden;

$M^5$ $-CH_2-O-$, -CO-O-, $-O-CH_2-$, -O-CO- oder eine Einfachbindung;

k, l, m, n, o, p, q, r Null oder Eins, unter der Bedingung, daß die Summe k+m+p+r kleiner 4 und größer Null ist; mit der Maßgabe, daß

a) eine der beiden Gruppen $R^1$ und $R^2$ die Gruppe

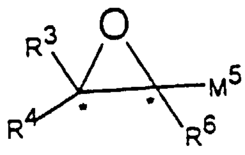

ist, oder

b) eine der Gruppen $A^1$ und $A^2$, $A^3$, $A^4$ 1,3-Dioxan-2,5-diyl,(1,3,4)-Thiadiazol-2,5-diyl oder 1,3-Dioxaborinan-2,5-diyl ist, oder

c) in einer der Gruppen $R^1$ und $R^2$ eine $-CH_2$-Gruppe durch $-Si(CH_3)$-ersetzt ist, oder

d) die Verbindung der Formel (I) ausgewählt ist aus

2-Fluor-6-(4-(4-octyloxyphenyl)phenyl)pyridin,
2-Fluor-3-octyloxy-6-(4-(4-octyloxyphenyl)phenyl)pyridin,
2-Fluor-3-hexyloxy-6-(4-(4-octylphenyl)phenyl)pyridin,
2-Fluor-3,6-di(4-octyloxyphenyl)pyridin,
2-Fluor-3-(4-heptylphenyl)-6(4-octyloxyphenyl)pyridin,
trans-4-Pentylcyclohexancarbonsäure-(2-fluor-6(4-octyloxyphenyl) pyridin-3-yl)ester,
2-Fluor-3-(4-(4-octylphenyl)phenyl)-6-(4-octyloxyphenyl)pyridin,
2-Fluor-3-octyloxy-6-[4-(4-octylphenyl)phenyl]pyridin.

**2.** Fluorpyridinverbindungen nach Anspruch 1, ausgewählt aus

3(4-(Butyldimethylsilyl)butoxy-2-Fluor-6-(4-(trans-4-pentylcyclohexyl)phenyl)pyridin,
2-Fluor-3-(5-octyl-1,3-dioxaborinan-2-yl)-6-(4-octyloxyphenyl)pyridin,
2-Fluor-3-(5-octyl-1,3-dioxan-2-yl)-6-(4-octyloxyphenyl)pyridin,
((2S,3S)-3-Pentyloxiran-2-yl)methyl-(2-fluor-6-(4-octyloxyphenyl)pyridin-3-yl)ether,
((2S,3S)-3-Pentyloxiran-2-yl)methyl-(4-(2-fluor-3-octyloxypyridin-6-yl) phenyl)ether,
((2S,3S)-3-Pentyloxiran-2-yl)methyl-(2-fluor-6-(4-(trans-4-pentylcyclohexyl)phenyl)pyridin-3-yl)ether.

**3.** Flüssigkristalline Mischung, enthaltend mindestens eine Fluorpyridinverbindung der Formel (I) gemaß Anspruch 1 und/oder 2.

**4.** Flüssigkristalline Mischung nach Anspruch 3, dadurch gekennzeichnet, daß sie aus 2 bis 20 Komponenten besteht.

**5.** Flüssigkristalline Mischung nach Anspruch 3 oder 4, dadurch gekennzeichnet, daß sie 0,1 bis 70 Mol-% an einem oder mehreren Fluorpyridinen der Formel (I) gemäß Anspruch 1 und/oder 2 enthält.

**6.** Flüssigkristalline Mischung nach einem oder mehreren der Ansprüche 3 bis 5, dadurch gekennzeichnet, daß sie ferroelektrisch ist.

**7.** Ferroelektrische Schalt- und Anzeigevorrichtung, enthaltend Trägerplatten, Elektroden, mindestens einen Polarisator, mindestens eine Orientierungsschicht sowie ein flüssigkristallines Medium, dadurch gekennzeichnet, daß das flüssigkristalline Medium eine Flüssigkristallmischung nach Anspruch 6 ist.

## Claims

1. A fluoropyridine compound of the formula I

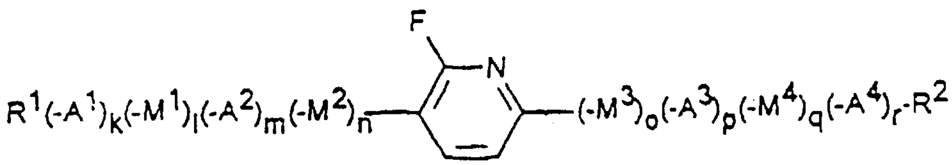

in which the symbols have the following meaning:

$R^1$, $R^2$, independently of one another, are H, F, CN or straight-chain or branched (with or without the inclusion of an asymmetric carbon atom) alkyl having 1 to 16 atoms, it also being possible for one $-CH_2-$ group to be replaced by -O-, -S-, -CO-, -CO-O-, -O-CO-, -O-CO-O-, -CH=CH-, -C≡C-, Δ or $-Si(CH_3)_2-$, or are one of the following chiral groups:

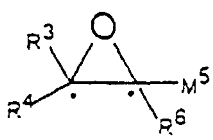 , 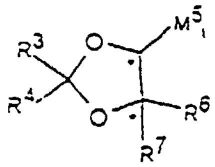 , 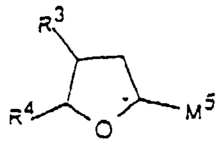 , 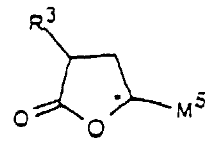

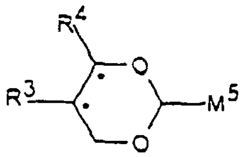 ,

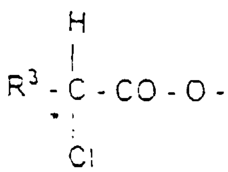, 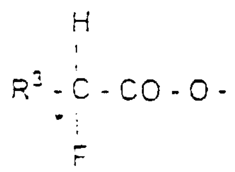, 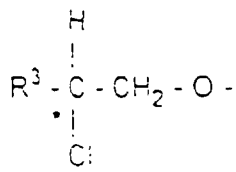, 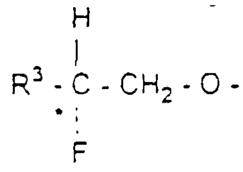

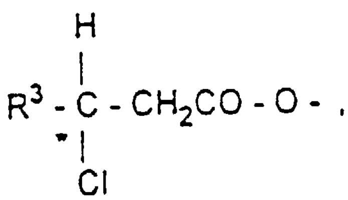, 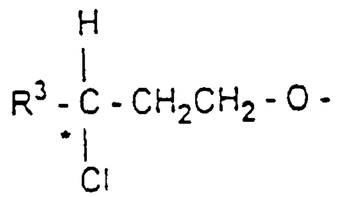,

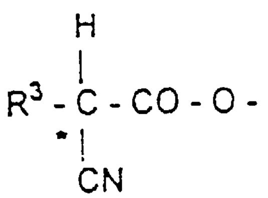, 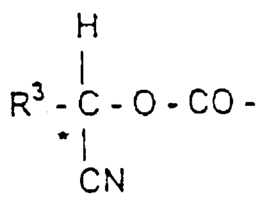

$A^1$, $A^2$, $A^3$, $A^4$, identical or different, are 1,4-phenylene, pyrazine-2,5-diyl, pyridazine-3,6-diyl, pyridine-2,5-diyl, pyrimidine-2,5-diyl, it being possible for one or two hydrogen atoms to be replaced by F, trans-1,4-cyclohexylene, 1,3,4-thiadiazole-2,5-diyl, 1,3-dioxane-2,5-diyl, naphthalene-2,6-diyl, bicyclo[2.2.2]octane-1,4-diyl or 1,3-dioxaborinane-2,5-diyl;

$M^1$, $M^2$, $M^3$, $M^4$, identical or different, are -O-, -CO-, -CO-O-, -O-CO-, $-CH_2-O-$, $-O-CH_2-$, $-CH_2-CH_2-$, -CH=CH- or -C≡C-;

$R^3$, $R^4$, $R^6$, $R^7$, independently of one another, are H or straight-chain or branched alkyl having 1 to 10 carbon atoms or $R^3$ and $R^4$ together are also $-(CH_2)_4-$or $-(CH_2)_5-$ if bound as substituents to a dioxolane system;

$M^5$ is $-CH_2-O-$, -CO-O-, $-O-CH_2-$, -O-CO- or a single bond;

k, l, m, n, o, p, q, r are zero or one, with the proviso that the sum of $k + m + p + r$ is less than 4 and greater than zero;
with the proviso that

a) one of the two groups $R^1$ and $R^2$ is the group

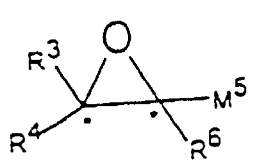

,

or
b) one of the groups $A^1$ and $A^2$, $A^3$, $A^4$ is 1,3-dioxane-2,5-diyl, 1,3,4-thiadiazole-2,5-diyl or 1,3-dioxaborinane-2,5-diyl, or
c) in one of the groups $R^1$ and $R^2$ a $CH_2$- group is replaced by $-Si(CH_3)_2-$, or
d) the compound of the formula (I) is selected from

2-fluoro-6-(4-(4-octyloxyphenyl)phenyl)-pyridine,

2-fluoro-3-octyloxy-6-(4-(4-octyloxyphenyl)-phenyl)pyridine,
2-fluoro-3-hexyloxy-6-(4-(4-octylphenyl)-phenyl)pyridine,
2-fluoro-3,6-di(4-octyloxyphenyl)pyridine,
2-fluoro-3-(4-heptylphenyl)-6-(4-octyloxyphenyl)pyridine,
2-fluoro-6-(4-octyloxyphenyl)-3-pyridinyl trans-4-pentylcyclohexanecarboxylate,
2-fluoro-3-(4-(4-octylphenyl)phenyl)-6-(4-octyloxyphenyl)pyridine, and
2-fluoro-3-octyloxy-6-[4-(4-octylphenyl)-phenyl]pyridine.

**2.** A fluoropyridine compound as claimed in claim 1, selected from

3-(4-(butyldimethylsilyl)butoxy-2-fluoro-6-(4-(trans-4-pentylcyclohexyl)phenyl)pyridine,
2-fluoro-3-(5-octyl-1,3-dioxaborinan-2-yl)-6-(4-octyloxyphenyl)pyridine,
(2S,3S)-3-pentyl-2-oxiranylmethyl 2-fluoro-6-(4-octyloxyphenyl)-3-pyridinyl ether,
(2S,3S)-3-pentyl-2-oxiranylmethyl 4-(2-fluoro-3-octyloxy-6-pyridinyl)phenyl ether,
(25,3S)-3-pentyl-2-oxiranylmethyl 2-fluoro-6-(4-(trans-4-pentylcyclohexyl)phenyl)-3-pyridinyl ether.

**3.** A liquid crystal mixture comprising at least one fluoropyridine compound of the formula (I) as claimed in claim 1 and/or 2.

**4.** A liquid crystal mixture as claimed in claim 3, which consists of from 2 to 20 components.

**5.** A liquid crystal mixture as claimed in claim 3 or 4, which comprises from 0.1 to 70 mol%, of one or more fluoropyridines of the formula (I) as claimed in claim 1 and/or 2.

**6.** A liquid crystal mixture as claimed in one or more of claims 3 to 5, which is ferroelectric.

**7.** A ferroelectric switching and display device, comprising base plates, electrodes, at least one polarizer, at least one orientation layer and a liquid-crystalline medium, wherein the liquid-crystalline medium is a liquid crystal mixture as claimed in claim 6.

**Revendications**

**1.** Composé fluoropyridine de formule générale I

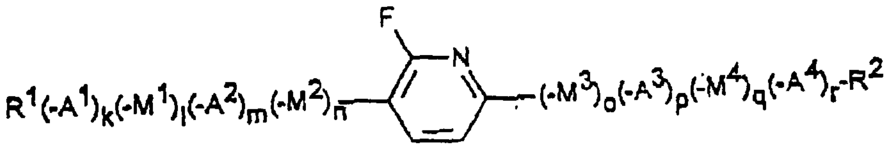

dans laquelle les symboles ont la signification suivante

$R^1$, $R^2$ représentent indépendamment l'un de l'autre H, F, CN ou un groupe alkyle linéaire ou ramifié (avec ou sans atome de carbone asymétrique) ayant 1 a 16 atomes de carbone, dans lequel également un groupe -$CH_2$- peut être remplacé par -O-, -S-, -CO-, -CO-O-, -O-CO-, -O-CO-O-, -CH=CH-, -C≡-C-, Δ ou - $Si(CH_3)_2$, ou un des groupes chiraux suivants :

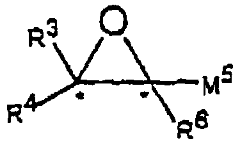 , 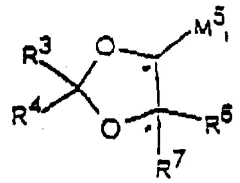 , 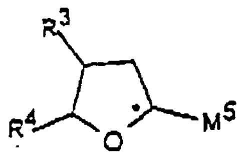 , 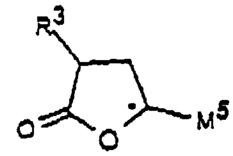

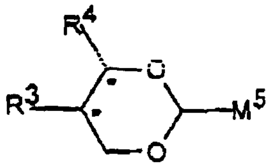 ,

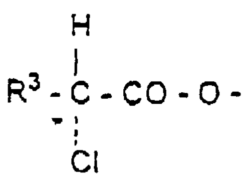 , 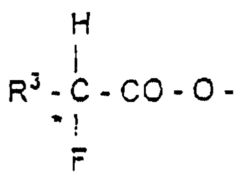 , 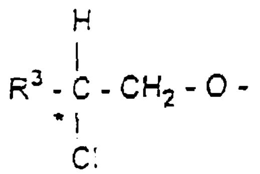 , 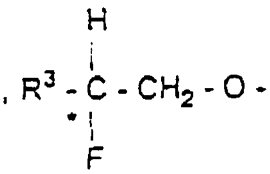

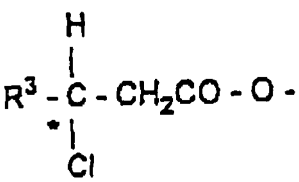 , 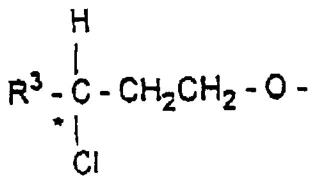 ,

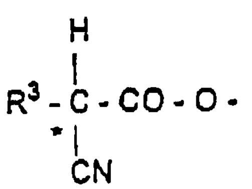 , 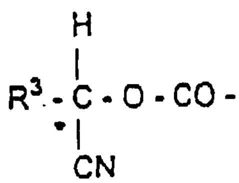

$A^1$, $A^2$, $A^3$, $A^4$, identiques ou différents représentent un groupe 1,4-phénylène, pyrazin-2,5-diyle, pyridazin-3,6-diyle, pyridin-2,5-diyle, pyrimidin-2,5-diyle, dans lequel un ou deux atomes d'hydrogène peuvent être remplacés par un atome de fluor, trans-1,4-cyclohexylène, (1,3,4)-thiadiazol-2,5-diyle, 1,3-dioxan-2,5-diyle, naphtalèn-2,6-diyle, bicyclo[2.2.2]octan-1,4-diyle ou 1,3-dioxaborinan-2,5-diyle;

$M^1$, $M^2$, $M^3$, $M^4$, identiques ou différents représentent -O-, -CO-, CO-O-, -O-CO-, $-CH_2-O-$, $-O-CH_2-$, $-CH_2-CH_2-$, -CH=CH- ou -C≡C-;

$R^3$, $R^4$, $R^6$, $R^7$, indépendamment les uns des autres représentent H ou un groupe alkyle linéaire ou ramifié avec 1 à 10 atomes de carbone ou $R^3$ et $R^4$ forment également ensemble $-(CH_2)_4-$ ou $-(CH_2)_5-$lorsqu'ils sont liés comme substituants à un système dioxolanne;

$M^5$ représente $-CH_2-O-$, -CO-O-, $-O-CH_2-$, -O-CO- ou une simple liaison;

k, l, m, n, o, p, q, r sont zéro ou un, à la condition, que la somme $k + m + p + r$ soit inférieure à 4 et supérieure à zéro;

à la condition que

a) un des deux groupes $R^1$ et $R^2$ soit le groupe

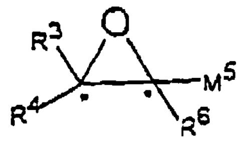

,

ou

b) un des groupes $A^1$ et $A^2$, $A^3$, $A^4$ soit un groupe 1,3-dioxan-2,5-diyle, (1,3,4)-thiadiazol-2,5-diyle ou 1,3-dioxaborinan-2,5-diyle, ou

c) dans un des groupes $R^1$ et $R^2$ un groupe $-CH_2-$ soit remplacé par $-Si(CH_3)_2-$, ou

d) le composé de formule (I) soit choisi parmi

la 2-fluoro-6-(4-(4-octyloxyphényl)phényl)pyridine,
la 2-fluoro-3-octyloxy-6-(4-(4-octylophényl)phényl)pyridine,
la 2-fluoro-3-hexyloxy-6-(4-(4-octylphényl)phényl)pyridine,
la 2-fluoro-3,6-di(4-octyloxyphényl)pyridine,
la 2-fluoro-3-(4-heptylphényl)-6-(4-octyloxyphényl)pyridine,
le trans-4-pentylcyclohexanecarboxylate de 2-fluoro-6-(4-octyloxyphényl)pyridin-3-yle,
la 2-fluoro-3-(4-(4-octylphényl)phényl)-6-(4-octyloxyphényl)pyridine,
la 2-fluoro-3-octyloxy-6-[4-(4-octylphényl)phényl]pyridine.

**2.** Composés fluoropyridine selon la revendication 1, choisis parmi

la 3(4-butyldiméthylsilyl)butoxy-2-fluoro-6-(4-(trans-4-pentylcyclohexyl)phényl)-pyridine,
la 2-fluoro-3-(5-octyl-1,3-dioxaborinan-2-yl)-6-(4-octyloxyphényl)pyridine,
la 2-fluoro-3-(5-octyl-1,3-dioxan-2-yl)-6-(4-octyloxyphényl)pyridine,
le ((2S,3S)-3-pentyloxiran-2-yl)méthyl-2-fluoro-6-(4-octyloxyphényl)pyridin-3-yl]éther,
le ((2S,3S)-3-pentyloxiran-2-yl)méthyl-(4-(2-fluoro-3-octyloxypyridin-6-yl)phényl)éther,
le ((2S,3S)-3-pentyloxiran-2-yl)méthyl-(2-fluoro-6-(4-trans-4-pentylcyclohexyl) phényl)pyridin-3-yl)éther.

**3.** Composition de cristaux liquides contenant au moins un composé fluoropyridine de formule (I) selon la revendication 1 et/ou 2.

**4.** Composition de cristaux liquides selon la revendication 3, caractérisée en ce qu'elle est constituée de 2 à 20 composants.

**5.** Composition de cristaux liquides selon la revendication 3 ou 4, caractérisée en ce qu'elle contient 0,1 à 70% en mole d'un ou de plusieurs fluoropyridines de formule (I) selon la revendication 1 et/ou 2.

**6.** Composition de cristaux liquides selon une ou plusieurs des revendications 3 à 5, caractérisée en ce qu'elle est ferroélectrique.

**7.** Dispositif ferroélectrique de commutation et d'affichage, contenant des cartes support, des électrodes, au moins un polariseur, au moins une couche d'orientation et un mélange à cristal liquide, caractérisé en ce que le milieu à cristal liquide est une composition de cristaux liquides selon la revendication 6.